# EUROPEAN PATENT SPECIFICATION

(11) **EP 2 734 622 B1**
(45) Date of publication and mention of the grant of the patent: **05.09.2018**
(21) Application number: 12814129.8
(22) Date of filing: 19.07.2012
(51) Int. Cl.: C07K 14/00, C12N 5/077, A61K 38/16, A61L 27/38, C07K 19/00

(54) **COMPOSITIONS AND METHODS FOR RE-PROGRAMMING CELLS WITHOUT GENETIC MODIFICATION FOR REPAIRING CARTILAGE DAMAGE**
ZUSAMMENSETZUNGEN UND VERFAHREN ZUR UMPROGRAMMIERUNG VON ZELLEN OHNE GENETISCHE MODIFIKATION ZUR REPARATUR VON KNORPELSCHÄDEN
COMPOSITIONS ET PROCÉDÉS DE REPROGRAMMATION DE CELLULES SANS MODIFICATION GÉNÉTIQUE POUR LA RÉPARATION DE CARTILAGES ENDOMMAGÉS

(30) Priority: 19.07.2011 US 201161509114 P
(43) Date of publication of application: 28.05.2014
(73) Proprietor: Vivoscript, Inc., Costa Mesa, CA 92626-1431 (US); Oregon Health & Science University, Portland, OR 97239 (US)
(72) Inventor: ZHU, Yong, Anaheim, California 92807 (US); WU, Shili, Edmond, Oklahoma 73012 (US); BAO, Jun, Rowland Heights, California 91748 (US); CHU, Cong-Qiu, Portland, Oregon 97239 (US)
(74) Representative: Goodfellow, Hugh Robin
(86) International application number: PCT/US2012/047495
(87) International publication number: WO 2013/013105

(56) References cited:
- EP-A1- 2 377 926
- WO-A1-2010/075575
- WO-A1-2010/075575
- WO-A1-2011/050334
- WO-A1-2011/050476
- WO-A2-2010/059806
- WO-A2-2011/097181
- WO-A2-2011/139688
- W. HUANG ET AL: "Phosphorylation of SOX9 by Cyclic AMP-Dependent Protein Kinase A Enhances SOX9's Ability To Transactivate a Col2a1 Chondrocyte-Specific Enhancer", MOLECULAR AND CELLULAR BIOLOGY, vol. 20, no. 11, 1 June 2000 (2000-06-01), pages 4149-4158, XP055154002, ISSN: 0270-7306, DOI: 10.1128/MCB.20.11.4149-4158.2000
- T. HATTORI ET AL: "Interactions between PIAS Proteins and SOX9 Result in an Increase in the Cellular Concentrations of SOX9", JOURNAL OF BIOLOGICAL CHEMISTRY, vol. 281, no. 20, 19 May 2006 (2006-05-19) , pages 14417-14428, XP055154011, ISSN: 0021-9258, DOI: 10.1074/jbc.M511330200
- EKATERINA Y. LUKIANOVA-HLEB ET AL: "Plasmonic Nanobubbles Enhance Efficacy and Selectivity of Chemotherapy Against Drug-Resistant Cancer Cells", ADVANCED MATERIALS, vol. 24, no. 28, 24 July 2012 (2012-07-24) , pages 3831-3837, XP055161010, ISSN: 0935-9648, DOI: 10.1002/adma.201103550
- D. SANO ET AL: "Vandetanib Restores Head and Neck Squamous Cell Carcinoma Cells' Sensitivity to Cisplatin and Radiation In Vivo and In Vitro", CLINICAL CANCER RESEARCH, vol. 17, no. 7, 24 February 2011 (2011-02-24), pages 1815-1827, XP055161013, ISSN: 1078-0432, DOI: 10.1158/1078-0432.CCR-10-2120
- VICTORIA SEBBAGE.: 'Cell-penetrating peptides and their therapeutic applications' BIOSCIENCE HORIZONS. vol. 2, no. 1, 2009, pages 64 - 72, XP008163524
- TEW SIMON R ET AL: "SOX9 transduction of a human chondrocytic cell line identifies novel genes regulated in primary human chondrocytes and in osteoarthritis", ARTHRITIS RESEARCH AND THERAPY, BIOMED CENTRAL, LONDON, GB, vol. 9, no. 5, 12 October 2007 (2007-10-12), page R107, XP021041145, ISSN: 1478-6354
- JAMES J. CRONICAN ET AL: "Potent Delivery of Functional Proteins into Mammalian Cells in Vitro and in Vivo Using a Supercharged Protein", ACS CHEMICAL BIOLOGY, vol. 5, no. 8, 20 August 2010 (2010-08-20) , pages 747-752, XP055242284, US ISSN: 1554-8929, DOI: 10.1021/cb1001153

## Description

### BACKGROUND OF THE INVENTION

Embryonic stem cells are capable of differentiating into many types of cells of the human body. The majority of somatic cells are terminally differentiated and were believed to lack the capability of changing to other types of somatic cells. Recent advances in induced pluripotent stem cell (iPSC) and transdifferentiation fields have changed this paradigm. Somatic cells can be reprogrammed to induced pluripotent stem cell (iPSC), via the ectopic expression of four transcription factors: Oct4 (e.g. SEQ ID NO: 1), Sox2 (e.g. SEQ ID NO: 2), Klf4 (SEQ ID NO: 3), and cMyc (e.g. SEQ ID NO: 4) via viral transduction (Okita et al, Nature 448, 313-317 (2007); Takahashi and Yamanaka, Cell 126, 663-676 (2006)). A number of modified genetic approaches were further developed to produce iPSCs with potentially reduced risks, including using non-integrating adenoviruses to deliver reprogramming genes (Stadtfeld et al., Science 322, 945-949 (2008)), transient transfection of reprogramming plasmids (Okita et al., Science 322, 949-953 (2008)), a piggyBac transposition system and Cre-excisable viruses (Soldner et al., Cell 136, 964-977 (2009); Woltjen et al., Nature 458, 766-770 (2009)). Furthermore, strategies of exploiting endogenous gene expression in certain cell types also allowed easier reprogramming and/or with less required exogenous genes (Aasen et al., Nat Biotechnol 26, 1276-1284 (2008); Kim et al., Nature 454, 646-650 (2008); Shi et al., Cell Stem Cell 2, 525-528 (2008)). Moreover, small molecules have been identified that enhance reprogramming efficiency and replace certain reprogramming factors (Huangfu et al., Nat Biotechnol 26, 795-797 (2008); Huangfu et al., Nat Biotechnol 26, 1269-1275 (2008); Li et al., Cell Stem Cell 4, 16-19 (2009); Shi et al., Cell Stem Cell 3, 568-574 (2008); Shi et al., Cell Stem Cell 2, 525-528 (2008)). However, all of those methods to date still involve the use of genetic materials that may introduce unknown, unwanted, or even harmful genome modifications in exogenous sequences in target cells. Further, these methods lack adequate control over the expression levels of transgenes. There is a need in the field to reprogram cells without relying upon or introducing exogenous genetic materials.

Articular cartilage is a highly organized tissue with low cell density and limited nutrient supply. Once it is damaged by trauma or degenerative arthritis, it has a limited capacity for regeneration. The most common joint disorder, osteoarthritis (OA), afflicts millions of people with symptoms that include severe pain, swelling, and clicking of joints. Surgical methods have been designed to repair cartilage result in the production of fibrocartilage which temporarily alleviates pain but is not a long-term solution becausefibrocartilage has poor resistance to shear forces. Hyaline cartilage, on the other hand, provides shock absorption and lubrication in diarthrodial joints as articular cartilage. Hyaline cartilage's extracellular matrix, comprises collagen fibrils composed of types II, IX, and XI collagen molecules, proteoglycans, and other matrix proteins and is substantially durable compared to fibrocartilage. Thus, the goal for repair of cartilage injury is to regenerate organized hyaline cartilage. Healing of cartilage damage with hyaline cartilage rather than fibrocartilage remains a challenging clinical problem.

One surgical procedure called microfracture involves drilling small holes into the subchondral bone marrow space underlying regions of damaged cartilage. This induces bleeding at the defect site and induces the formation of a blood clot. The clot contains pluripotent mesenchymal stem cells (MSCs) from the bone marrow, which have the potential to differentiate into condroblasts and condrocytes. However, like other surgical procedures, the cartilage formed is fibro-cartilaginous.

WO 2010/075575 concerns compositions and methods for reprogramming biological samples without introducing exogenous genes.

WO2011/050334 concerns compositions and methods for reprogramming cells into brown fat cells, without the introduction of exogenous genes.

Huang et al. 2000. Mol Cell Biol. 4149-4158 concerns investigations into SOX9's ability to transactivate the *Col2a1* enhancer.

Hattori et al. 2006. J Biol Chem. 281:14417-14428 concerns investigations into the interactions between PIAS proteins and SOX9.

Tew et al. 2007. Arthritis Res Ther. 9, 5: 107 concerns the identification of novel genes regulated in human primary chondrocytes and in osteoarthritis.

Cronican et al. 2010 ACS Chem Biol. 8, 5: 747-752 concerns the delivery of proteins into mammalian cells using a supercharged protein.

EP2377926 (prior art under Article 54(3) EPC) concerns cells which enable the reproduction of cartilage tissue.

WO2011/097181 (prior art under Article 54(3) EPC) concerns compositions and methods for reprogramming cells to neurons, without the introduction of exogenous genes.

WO2010/059806 concerns methods for generating reprogrammed mammalian cells from differentiated cells, without using viral or plasmid vectors.

WO2011/050476 concerns methods for cell de-differentiation, transformation and eukaryotic cell reprogramming.

### BRIEF SUMMARY OF THE INVENTION

One aspect of the present invention relates to an *in vitro* method of reprogramming a mesenchymal stem cell (MSC), comprising: exposing the MSC to a transducible material comprising Sox9 polypeptide and a transduction domain linked to the Sox9 polypeptide covalently, non-covalently or via a linker, wherein the transduction domain is a supercharged GFP, and wherein the MSC is reprogrammed to change to a chondrogenic cell. The transducible material comprises Sox9 polypeptide which is capable of exerting reprogramming changes of a biological sample once transduced into a biological sample.

In certain embodiments of the invention, the supercharged GFP is covalently linked to the effector domain through a linker.

According to the invention, the transducible material is capable of becoming transducible in a specific environment surrounding a biological sample.

Another aspect of the present invention relates to a transducible material comprising Sox9 polypeptide and a transduction domain linked to the Sox9 polypeptide covalently, non-covalently or via a linker; wherein the transduction domain is a supercharged GFP; for use in therapy to reprogram an MSC, comprising: exposing the MSC to the transducible material; wherein the MSC is reprogrammed to change to a chondrogenic cell.

Another aspect of the present invention relates to a transducible material comprising Sox9 polypeptide and a transduction domain linked to the Sox9 polypeptide covalently, non-covalently or via a linker; for use in therapy by treating a condition involving cartilage damage in a biological organism, wherein the transduction domain is a supercharged GFP, optionally wherein the condition involving cartilage damage is joint disorder, osteoarthritis, cartilage injury, traumatic rupture or detachment, achondroplasia, costochondritis, spinal disc herniation, relapsing polychonritis, tumor, chondroma, chondrosarcoma, or peomorphic adenoma.

Another aspect of the present invention relates to an "in vitro" method of developing cell-based therapies for diseases or conditions involving cartilage damage comprising: reprogramming an MSC to a transplantable chondrogenic cell by exposing the MSC to a transducible material comprising Sox9 polypeptide and a transduction domain linked to the Sox9 polypeptide covalently, non-covalently or via a linker, wherein the transduction domain is a supercharged GFP; transplanting the transplantable chondrogenic cell into a biological sample; and assessing the therapeutic effects of the transplantable chondrogenic cell;

Another aspect of the present invention relates to a transducible material comprising Sox9 polypeptide and a transduction domain linked to the Sox9 polypeptide covalently, non-covalently or via a linker; for use in therapy to reprogram an MSC, in a method of developing cell-based therapies for diseases or conditions involving cartilage damage comprising: reprogramming the MSC to a transplantable chondrogenic cell by exposing the MSC to the transducible material; transplanting the transplantable chondrogenic cell into a biological organism; and assessing the therapeutic effects of the transplantable chondrogenic cell, wherein the transduction domain is a supercharged GFP.

### BRIEF DESCRIPTION OF THE DRAWINGS

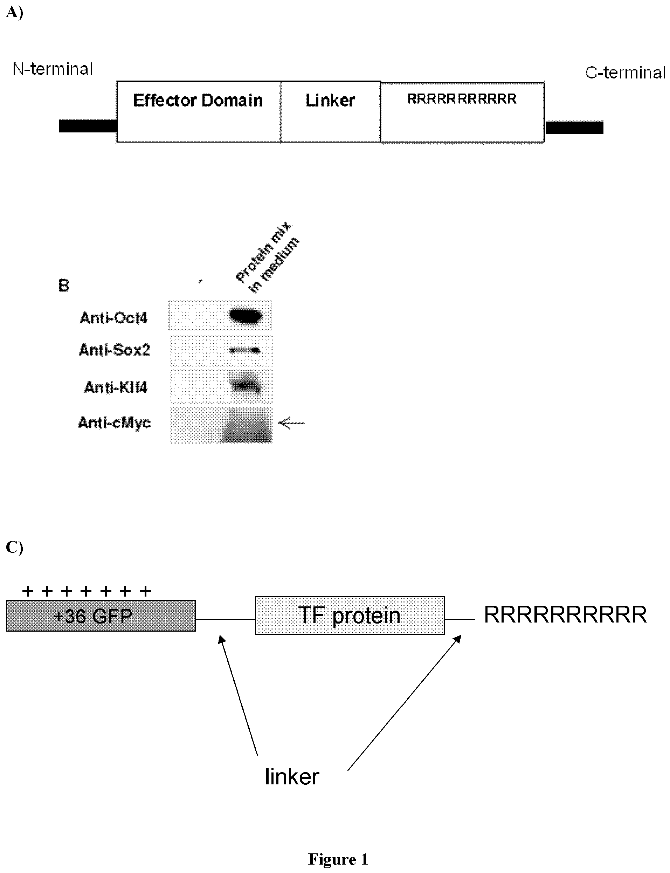

Figure 1: Characterization of transducible materials (I). (A) Schematic of protein expression vector for transducible materials 0ct4-11R (SEQ ID NO: 12), Sox2-11R (SEQ ID NO: 13), Klf4-11R (SEQ ID NO: 14), and cMyc-11R (SEQ ID NO: 15), Linker: SEQ ID NO: 55; Effector Domain: Oct4 (SEQ ID NO: 1), Sox2 (SEQ ID NO: 2), Klf4 (SEQ ID NO: 3), or cMyc (SEQ ID NO: 4). (B) Stability of the four transducible materials (Oct4-11R, Sox2-11R, Klf4-11R, and cMyc-11R) under the cell culture condition examined by Western blot analysis. (C) Schematic presentation of transducible materials containing super-charged green fluorescent protein (scGFP) fused to a transcription factor (TF).
Figure 2: Characterization of transducible materials (II). Protein transduction of 11R-tagged transducible materials into OG2-MEF cells examined by immunocytochemistry. Oct4: MEF cells transuded with Oct4-11R (green), Sox2: MEF cells transuded with Sox2-11R (red), Klf4: MEF cells transuded with K1f4-11R (red) and cMyc: MEF cells transuded with cMyc-11R (green). DAPI: Cells stained with DAPI to visualize the nuclei (blue) and the images were merged.
Figure 3: Characterization of transducible materials (III). Protein induced pluripotent stem (piPS) cells clonally expanded and self-renewed in chemical defined media and feeder free condition.

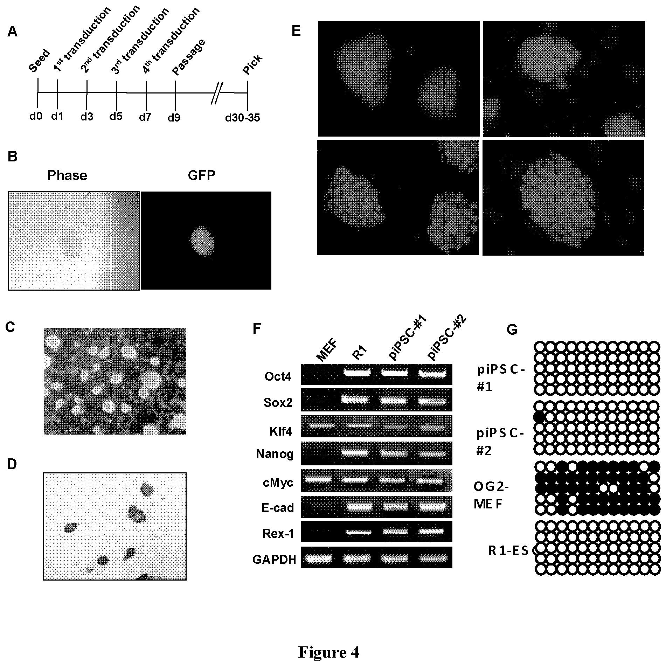

Figure 4: Generation of piPS cells by transducible materials Oct4-11R, Sox2-11R, K1f4-11R, and cMyc-11R. (A) Timeline of piPS cell generation. (B) Oct4-GFP⁺ piPS cell colonies initially observed around day 30-35. Phase: representative phase contrast image; and GFP: fluorescence image. (C) Oct4-GFP⁺ piPS cells sustained long term self-renewal under conventional mESC growth condition. (D) The long-term expanded piPS cells grew as compact and domed colonies that expressed strong ALP, a typical pluripotency marker. (E) piPS cells expressed other typical pluripotency markers, examined by immunofluorescence: SEA-1 (red), Sox2 (red), Oct4 (ed) and Nanog (red). DAPI: DAPI staining for visualization of the nuclei (blue), and the images were merged. (F) RT-PCR analysis of endogenous pluripotency gene expression in piPS cells. (G) Oct4 promoter methylation analysis by bisulfite genomic sequencing. Open and closed circles indicate unmethylated and methylated CpGs, respectively.
Figure 5: *In vitro* and *in vivo* pluripotency of piPS cells (I). piPS cells effectively differentiated *in vitro* into cells in the three germ layers: Tuj1: characteristic TUJ1⁺ neuronal cells-ectoderm (red): Bryt: Brachyury⁺ mesoderm cells (red); and Sox17: Sox17⁺ definitive endoderm cells. Images were merged with DAPI (blue) staining.
Figure 6: *In vitro* and *in vivo* pluripotency of piPS cells (II) (A) RT-PCR analysis of *in vitro* differentiation of piPS cells. (B) Chimeric embryos (13.5 dpc, 2 out of 7, left) obtained after transfer of the piPS cell aggregated embryos into a pseudo-pregnant mouse (top). Such piPS cells contributed to the germline cells (Oct4-GFP positive) in isolated genital ridge tissue from chimeric embryos (bottom).
Figure 7: Schematic of protein expression vectors for transducible materials. His6: SEQ ID NO: 59; Effector Domain: Ngn3 (SEQ ID NO: 8), PDX1 (SEQ ID NO: 9); MafA (SEQ ID NO: 10), or Foxp3 (SEQ ID NO: 11); Linker: SEQ ID NO: 55.
Figure 8: Reprogramming of liver and pancreatic exocrine cells to insulin-producing beta cells by transducible materials His6-Ngn3-11R (SEQ ID NO: 30), His6-PDX1-11R (SEQ ID NO: 31) and His6-MafA-11R (SEQ ID NO: 32) in mouse (I). Mouse-1, Mouse-2 and Mouse-3 were treated with bovine serum albumin (BSA) protein (control group). Mouse-4, Mouse-5 and Mouse-6 were treated with His6-Ngn3-11R, His6-PDX1-11R and His6-MafA-11R (treatment group). A) Immunofluorescent analysis (IFA) of Mouse-1 liver; B) IFA of Mouse-2 liver; and C) IFA of Mouse-3 liver.
Figure 9: Reprogramming of liver and pancreatic exocrine cells to insulin-producing beta cells by transducible materials His6-Ngn3-11R, His6-PDX1-11R and His6-MafA-11R in mouse (II). Mouse-1, Mouse-2 and Mouse-3 were treated with bovine serum albumin (BSA) protein (control group). Mouse-4, Mouse-5 and Mouse-6 were treated with His6-Ngn3-11R, His6-PDX1-11R and His6-MafA-11R (treatment group). A) IFA of Mouse-4 liver (1); B) IFA of Mouse-4 liver (2); C) IFA of Mouse-5 liver (1); D) IFA of Mouse-5 liver (2); E) IFA of Mouse-6 liver (1); and F) IFA of Mouse-6 liver (2).
Figure 10: Reprogramming of liver and pancreatic exocrine cells to insulin-producing beta cells by transducible materials His6-Ngn3-11R, His6-PDX1-11R and His6-MafA-11R in mouse (III). Mouse-1, Mouse-2 and Mouse-3 were treated with bovine serum albumin (BSA) protein (control group). Mouse-4, Mouse-5 and Mouse-6 were treated with His6-Ngn3-11R, His6-PDX1-11R and His6-MafA-11R (treatment group). A) IFA of Mouse-1 pancrease; B) IFA of Mouse-2 pancrease (1); C) IFA of Mouse-2 pancrease (2); and D) IFA of Mouse-3 pancrease.
Figure 11: Reprogramming of liver and pancreatic exocrine cells to insulin-producing beta cells by transducible materials His6-Ngn3-11R, His6-PDX1-11R and His6-MafA-11R in mouse (IV). Mouse-1, Mouse-2 and Mouse-3 were treated with bovine serum albumin (BSA) protein (control group). Mouse-4, Mouse-5 and Mouse-6 were treated with His6-Ngn3-11R, His6-PDX1-11R and His6-MafA-11R (treatment group). A) IFA of Mouse-4 pancrease (1); B) IFA of Mouse-4 pancrease (2); C) IFA of Mouse-5 pancrease (1); D) IFA of Mouse-5 pancrease (2); and E) IFA of Mouse-6 pancrease.
Figure 12: Reprogramming of T cells to Treg cells by transducible material His6-Foxp3-11R (SEQ ID NO: 33) (IA). Flow cytometric analysis of CD4 and CD25 protein expression in PBMC with lacking of CD14 monocytes: isotope control, PBS control, sample buffer control and protein (BSA 100µg/ml) control.
Figure 13: Reprogramming of T cells to Treg cells by transducible material His6-Foxp3-11R (IB). Flow cytometric analysis of CD4 and CD25 protein expression in PBMC with lacking of CD14 monocytes treated with His16-Foxp3-11R of 10µg/ml, 20 µg/ml, or 50 µg/ml.
Figure 14: Reprogramming of T cells to Treg cells by transducible material His6-Foxp3-11R (IIA). Flow cytometric analysis of CD4 and CD25 protein expression in PBMC: isotope control, and PBS control.
Figure 15: Reprogramming of T cells to Treg cells by transducible material His6-Foxp3-11R (IIB). Flow cytometric analysis of CD4 and CD25 protein expression in PBMC: sample buffer control and protein (BSA 100µg/ml) control.
Figure 16: Reprogramming of T cells to Treg cells by transducible material His6-Foxp3-11R (IIC). Flow cytometric analysis of CD4 and CD25 protein expression in PBMC treated with His16-Foxp3-11R of 10µg/ml, or 50 µg/ml.
Figure 17: Reprogramming of T cells to Treg cells by transducible material His6-Foxp3-11R (IID). Flow cytometric analysis of CD4 and CD25 protein expression in PBMC treated with His16-1R of 100 µg/ml.
Figure 18: Sequences of the exemplary effector domains of Foxo1 and Foxo3.

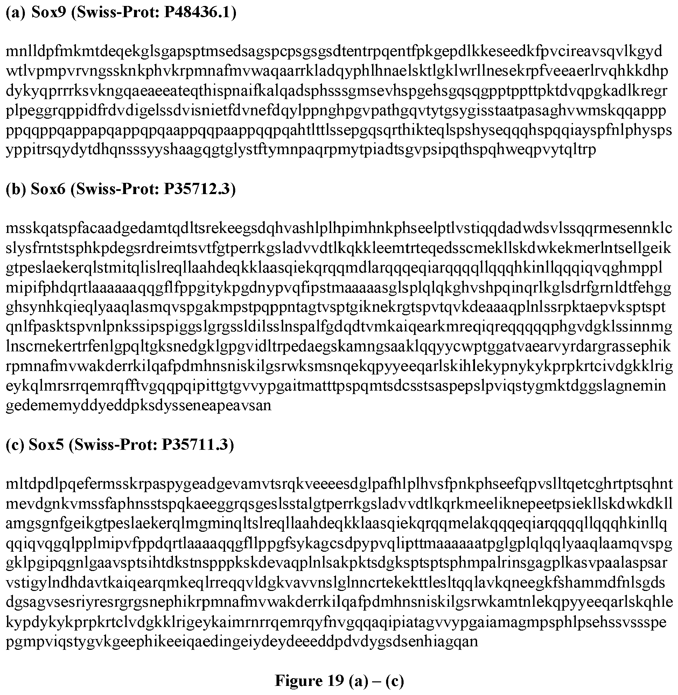

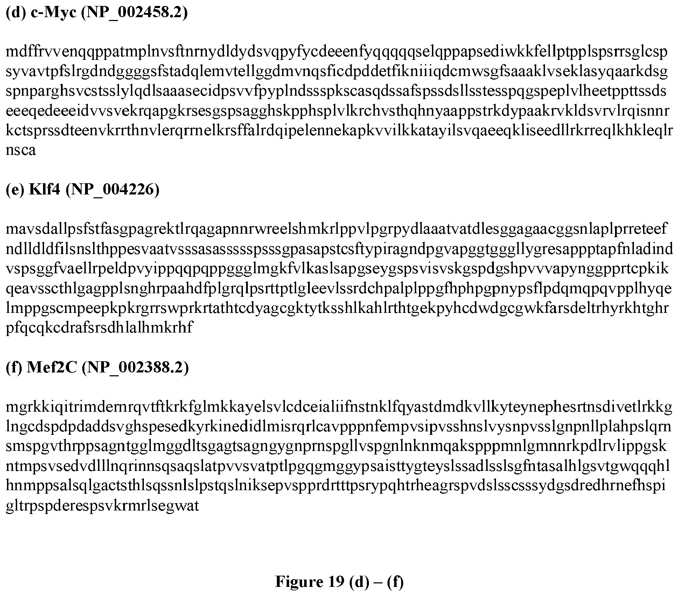

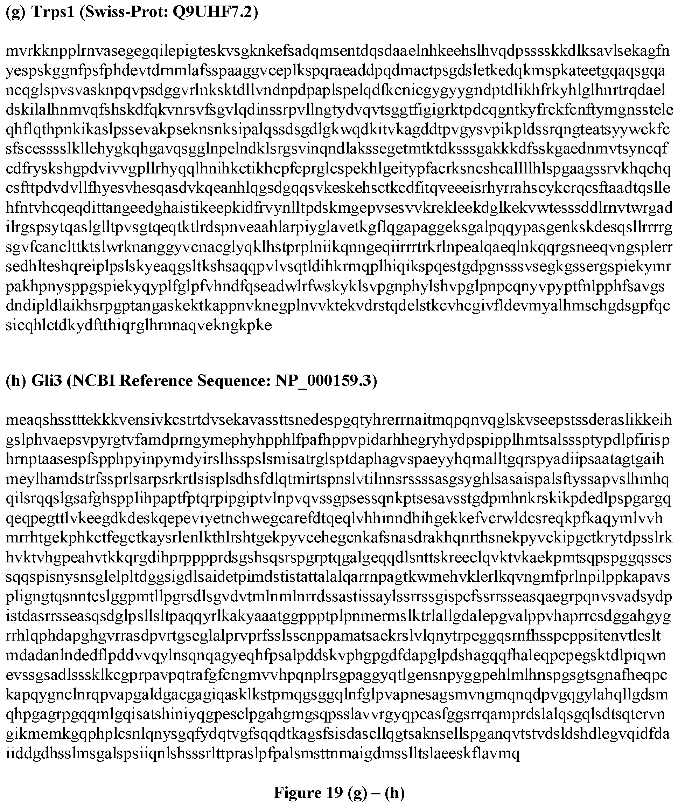

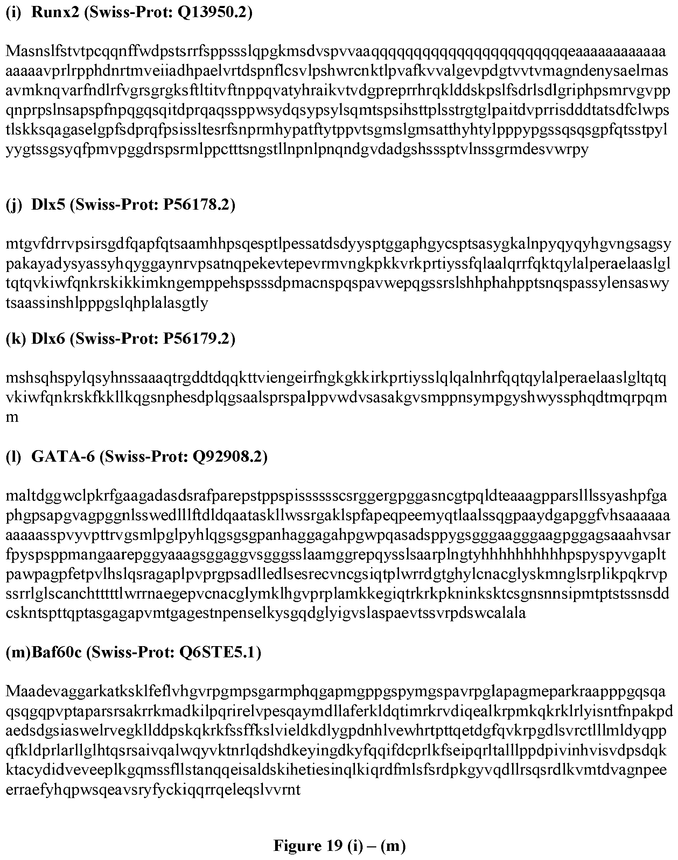

Figure 19: Sequences of the exemplary effector domains.

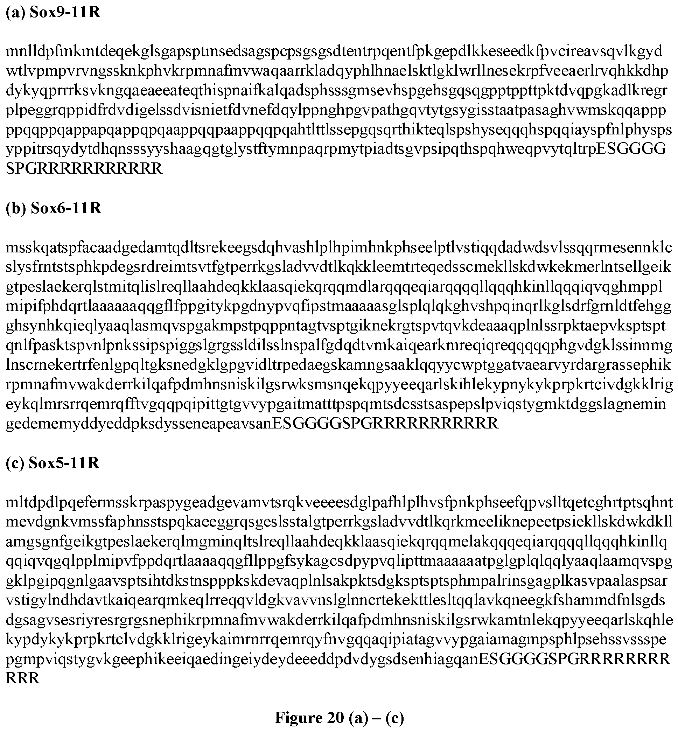

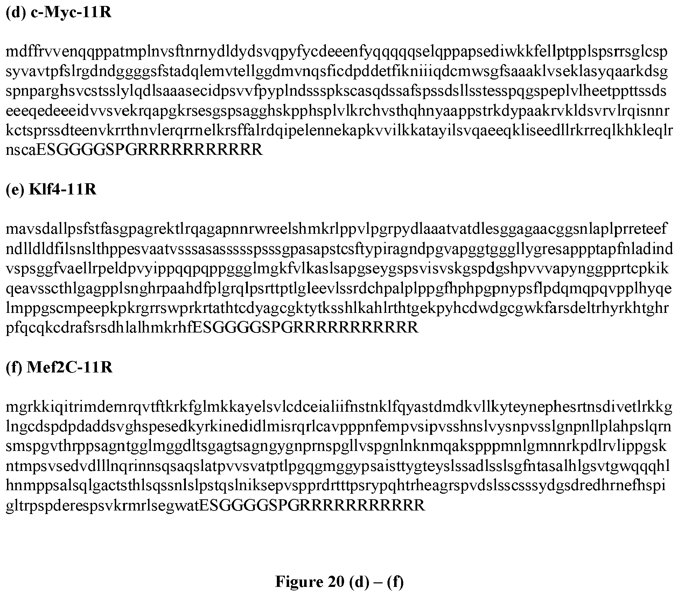

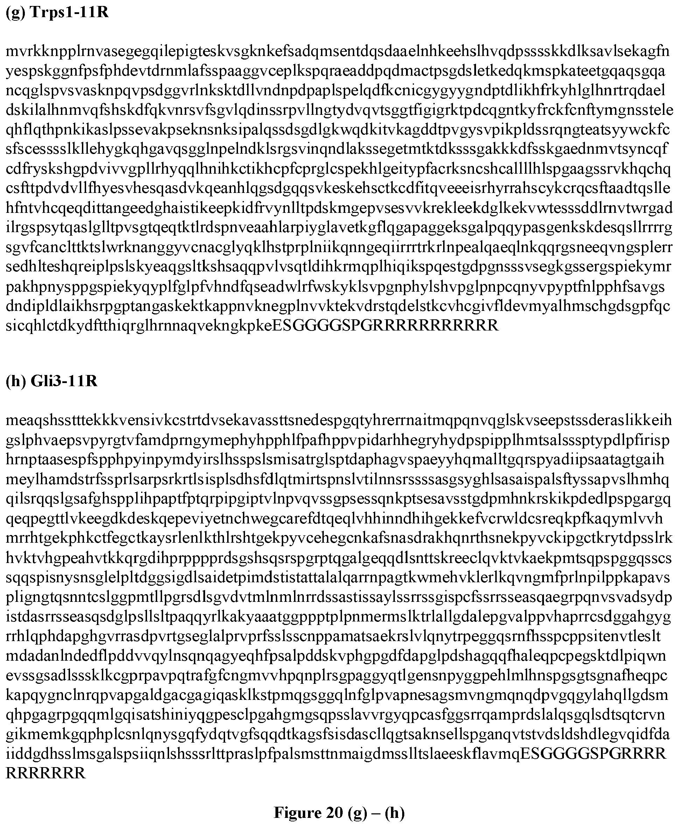

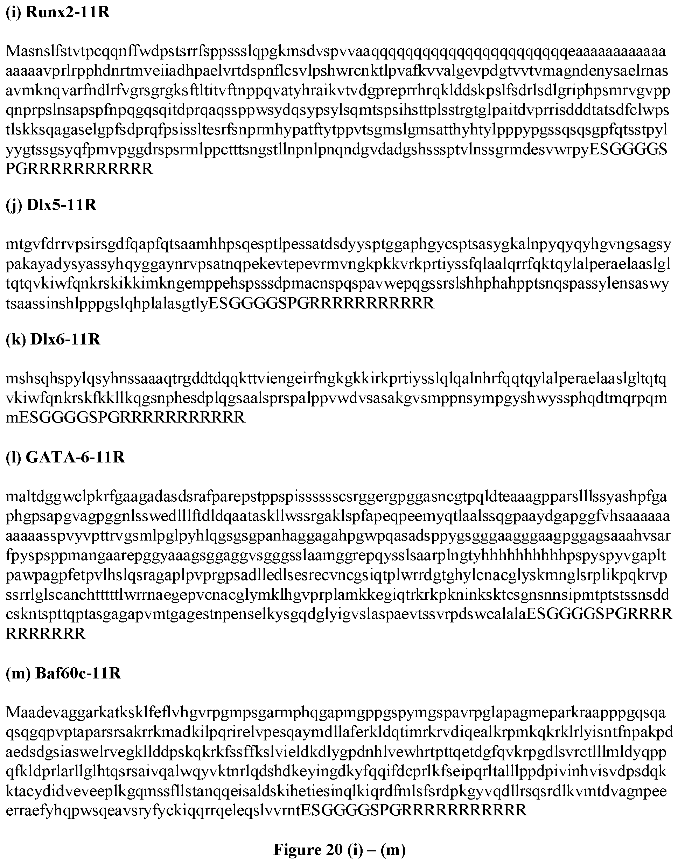

Figure 20: Sequences of the exemplary transducible materials: Effector domain-11R.

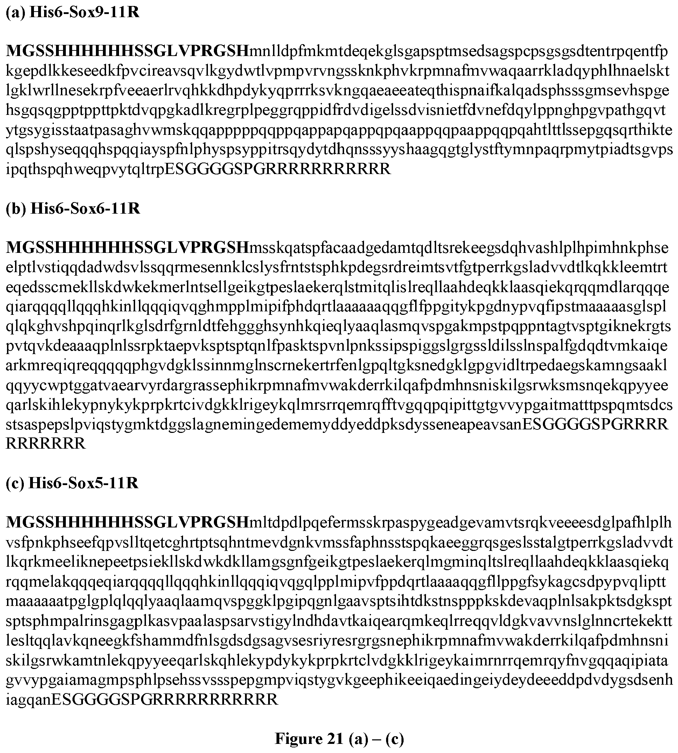

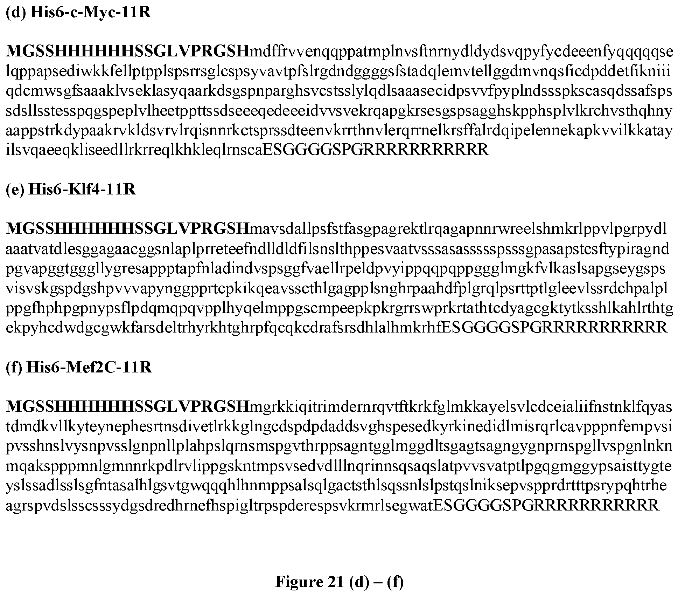

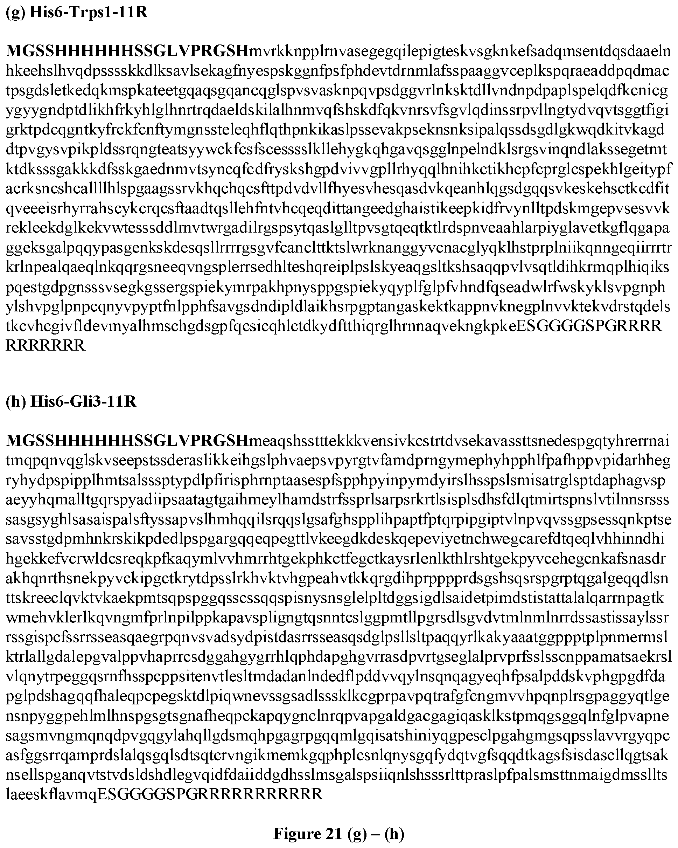

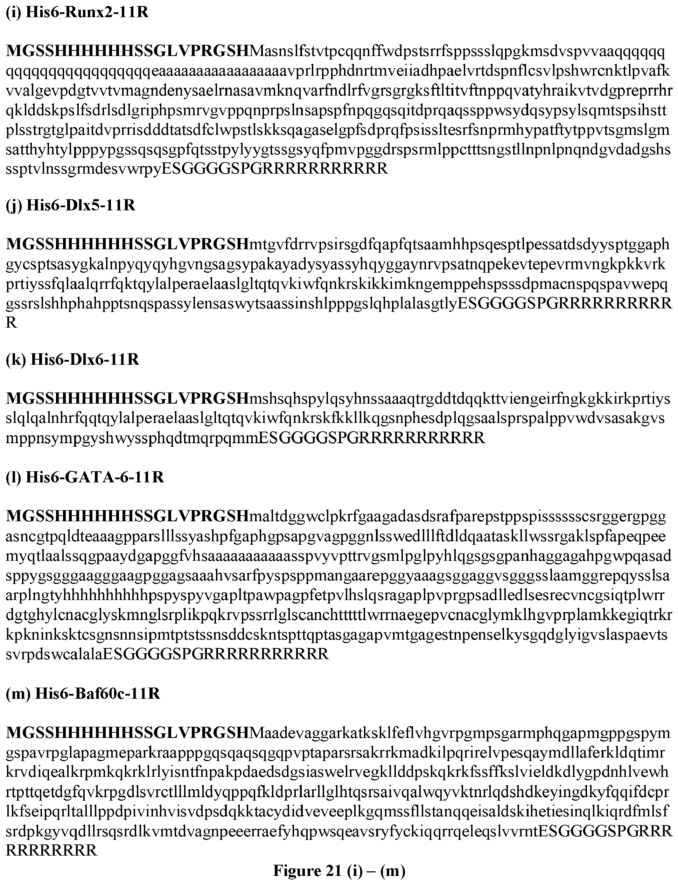

Figure 21: Sequences of the exemplary transducible materials: His6-Effector domain-11R.
Figure 22: Sequences of the exemplary transducible materials: HA2-supercharged GFP-Effector domain (a-m), and scGFP - Sox9 - 11R (n).
Figure 23: Penetration of scSox9 protein into human skin fibroblast cell line (HHF, A and B) and human bone marrow derived mesenchymal stem cells (MSC, C and D). Cells were incubated with 10 µg/ml of super charged green fluorescence protein (scGFP, A and C) or recombinant Sox9-scGFP fusion protein (scSox9, B and D).
Figure 24: scSox9 induced increased proliferation of MSC. MSC were cultured in DMEM containing 10% FBS and low glucose (1.5g/l) with addition of scSox9 or buffer only for 48 hours.
Figure 25: scSox9 induced aggrecan expression and chondrogenesis, as shown by positive toluidine blue staining of MSC treated with scSox9 at 3 days (B), and MSC with scSox9 at 14 days (C), in contrast to negative toluidine blue staining of MSC with buffer (A).
Figure 26: scSox9 induced collagen type II expression, as shown by positive collagen type II monoclonal antibody (immunoperoxidase) staining of MSC treated with scSox9 (B and D), in contrast to negative staining of MSC treated with scGFP (A and C).
Figure 27: scSox9 induced increased collagen type II but decreased collagen type I and type X expression. At the indicated time point, RNA was extracted from MSCs treated with buffer only or 10 µg/ml of scSox9, and RT-PCR was performed for collagen (Col) type I, II and X mRNA expression. Expression as relative to GAPDH.

### DETAILED DESCRIPTION OF THE INVENTION

One aspect of the present disclosure relates to a transducible material for reprogramming a biological sample to a chondrogenic sample. The reprogrammed chondrogenic sample can repair cartilage injury or damage by regenerating or producing cartilage, preferably hyaline cartilage. The transducible material comprises an effector domain.

In certain embodiments of the disclosure, a transducible material used herein refers to a material or a molecule which is not DNA or derived from DNA but is capable of crossing or transducing or being crossed through a membrane of a biological sample (e.g., a cell membrane) so that the transducible material can enter or be brought into the inside of the biological sample from the outside of the biological sample and exerts reprogramming efforts. For example, the transducible material may interact with cell-surface receptors which facilitate the entry of the material into cells through receptor mediated endocytosis.

In certain embodiments of the disclosure, a transducible material is a selective transducible material which is more likely to transduce into a specific type of biological samples (e.g. cancer or tumor cells) or becomes transducible in a specific microenvironment in or around a biological sample (e.g. microenvironment around cancer or tumor) than other biological samples. For example, according to the invention, the selective transducible material comprises a transduction domain (e.g. a cell-targeting peptide or an activatable cell penetrating peptide) that preferably delivers the selective transducible material into a specific type of biological sample or become transducible in a microenvironment around a biological sample.

Without being bounded to any theories, it is contemplated that the transducible materials may cross a cell membrane and enter into cytoplasm to reprogram cytoplasm activities such as translation, post-translation modification, signaling pathway, or the apoptosis pathway. It is further contemplated that the transducible material may cross the nucleus membrane and reprogram or modulate DNA or chromosomal replication, gene transcription, and RNA splicing.

An effector domain is a motif or a molecule which, once inside a biological sample, is capable of exerting reprogramming changes of the biological sample. The effector domain may interact with molecules (e.g., proteins, DNA, RNA, sugars, and lipids) in the biological sample (e.g., in cytoplasm or nuclei) and lead to changes such as proliferation, differentiation, dedifferentiation, transdifferentiation, retrodifferentiation, transdetermination, apoptosis, and morphogenesis. The effector domain can be 1) a polypeptide, or a fragment or a mimic thereof; 2) a polynucleotide which cannot be gene expressed once transduced or incorporated into the genome of the biological sample or cause genetic modification but nevertheless interacts with molecules in the biological sample (e.g., a ribozyme, an antisense molecule, a siRNA or miRNA, an oligonucleotide, and the like); and 3) a small molecule or other chemical compound (e.g. chemotherapy drugs).

In certain embodiments of the disclosure, an effector domain is inherently transducible, e.g. PDX1 (e.g. SEQ ID NO: 9) and NeuroD (e.g. SEQ ID NO: 7).

One example of the effector domain is part or the entirety of a polypeptide such as a transcription factor, a chromosome remodeling protein, an antibody, or a fragment or mimic thereof. Another example of the effector domain is a small molecule which is not a polymer and binds with a biopolymer such as protein, nucleic acid, or polysaccharide and alters the activity or function of the biopolymer. Examples of small molecules include, without limitation, acetylation inhibitors, transcription activators, signal pathway activators, signal pathway inhibitors, and methylation inhibitors.

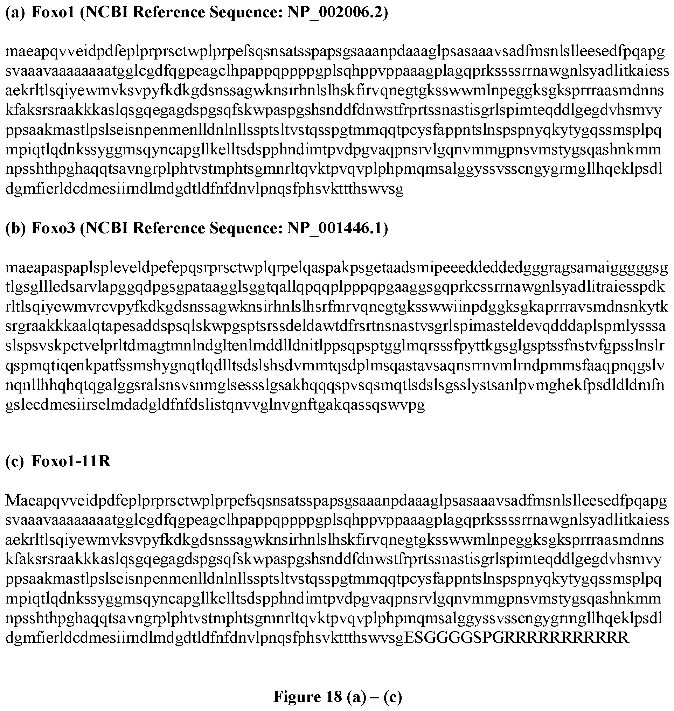

In another embodiment of the disclosure, an effector domain can be at least one polypeptide that reprograms a somatic or stem cell into a chondrogenic cell, reprograms a somatic cell into a stem cell or change a cell state from one to another. For example, the effector domain can be 1) a polypeptide selected from the group consisting of Klf4 (e.g. SEQ ID NO: 3), Sox2 (e.g. SEQ ID NO: 2), Lin28 (e.g. SEQ ID NO: 5), Oct4 (e.g. SEQ ID NO: 1), cMyc (e.g. SEQ ID NO: 4), Nanog (e.g. SEQ ID NO: 6), and any combination thereof; 2) a polypeptide selected from the group consisting of Klf4, Sox2, Oct4, cMyc, and any combination thereof; 3) a polypeptide selected from the group consisting of Sox2, Oct4, Lin28, Nanog, and any combination thereof; 4) a polypeptide selected from the group consisting ofNgn3 (e.g. SEQ ID NO: 8), PDX1 (e.g. SEQ ID NO: 9), MafA (e.g. SEQ ID NO: 10), NeuroD (e.g. SEQ ID NO: 7), and any combination thereof; 5) a polypeptide comprising Foxp3 (e.g. SEQ ID NO: 11), Foxo1 (e.g. Figure 18a) and Foxo3 (e.g. Figure 18b); 6) a polypeptide selected from the group consisting of Oct4, Sox2, Klf4, Lin28, Nanog, cMyc, Ngn3, PDX1, MafA, NeuroD, Foxp3, Foxo1 and Foxo3, and any combination thereof; 7) a combination of polypeptides Oct4, Sox2, Klf4 and cMyc; 8) a combination of polypeptides Ngn3, PDX1 and MafA; 9) a polypeptide selected from the group consisting of Sox9, Sox6, Sox5, c-Myc, Klf4, Mef2C, Trps1, Gli3, Runx2, Dlx5, Dlx6, GATA-6 and Baf60c; 10) a polypeptide selected from the group consisting of Oct4, Sox2, Klf4, Lin28, Nanog, cMyc, Ngn3, PDX1, MafA, NeuroD, Foxp3, Foxo1, Foxo2, Sox9, Sox6, Sox5, c-Myc, Klf4, Mef2C, Trps1, Gli3, Runx2, Dlx5, Dlx6, GATA-6 and Baf60c, and any combination thereof; and 11) a first polypeptide selected from the group consisting of Sox9, Sox6, Sox5, c-Myc, Klf4, Mef2C, Trps1, Gli3, Runx2, Dlx5, Dlx6, GATA-6 and Baf60c, and any combination thereof; and a second polypeptide selected from the group consisting of Oct4, Sox2, Klf4, Lin28, Nanog, cMyc, Ngn3, PDX1, MafA, NeuroD, Foxp3, Foxo1 and Foxo3.

**Table 1 Exemplary sequences of effector domains**

| Effector Domain | Exemplary Accession Number (Database) | Exemplary Polypeptide Sequence |
|---|---|---|
| Sox9 | P48436.1 (Swiss-Prot) | Figure 19 (a) |
| Sox6 | P35712.3 (Swiss-Prot) | Figure 19 (b) |
| Sox5 | P35711.3 (Swiss-Prot) | Figure 19 (c) |
| c-Myc | SEQ ID NO: 4; NP_002458.2 (NCBI Reference Sequence) | Figure 19 (d) |
| Klf4 | SEQ ID NO: 3; NP_004226 (NCBI Reference Sequence) | Figure 19 (e) |
| Mef2C | NP_002388.2 (NCBI reference) | Figure 19 (f) |
| Trps1 | (Q9UHF7.2) (Swiss-Prot) | Figure 19 (g) |
| Gli3 | NP_000159.3 (NCBI Reference Sequence) | Figure 19 (h) |
| Runx2 | Q13950.2 (Swiss-Prot) | Figure 19 (i) |
| D1x5 | P56178.2 (Swiss-Prot) | Figure 19 (j) |
| D1x6 | P56179.2 (Swiss-Prot) | Figure 19 (k) |
| GATA-6 | Q92908.2 (Swiss-Prot) | Figure 19 (1) |
| Baf60c | Q6STE5.1 (Swiss-Prot) | Figure 19 (m) |

The polypeptides provided herein encompass their homologous sequences. A "homologous sequence" as used herein, refers to a polypeptide which shares sufficient percentage of identity in amino acid sequence with a reference polypeptide to which it is homologous. In certain embodiments of the disclosure, a homologous sequence shares at least 70%, at least 75%, at least 80%, at least 85%, at least 88%, at least 90%, at least 93%, at least 95%, at least 97%, at least 98%, or at least 99% of identity in amino acid sequence with one of the polypeptides provided herein useful as effector domain. The polypeptides having homologous sequences have substantially the same activity as the effector domains disclosed herein.

The percentage of identity in amino acid sequence can be determined as the percentage of amino acid residues in a candidate amino acid sequence that are identical to the amino acid residues in a reference amino acid sequence, after aligning the sequences and, if necessary, introducing gaps, to achieve the maximum number of identical amino acids. Conservative substitution of the amino acid residues may be considered or may not considered as identical residues. "Conservative substitution" as used herein refers to replacing an amino acid residue with another amino acid residue that has similar physiochemical properties (e.g., hydrophobicity and molecular bulk of the side chain).

Alignment for purposes of determining percent amino acid sequence identity can be achieved in various ways known in the art. For instance, alignment of amino acid sequences may be made using publicly available tools such as BLASTp (available on the website of U.S. National Center for Biotechnology Information (NCBI): http://blast.ncbi.nlm.nih.gov/Blast.cgi, see also, Altschul S.F. et al, J. Mol. Biol., 215:403-410 (1990); Stephen F. et al, Nucleic Acids Res., 25:3389-3402 (1997)), ClustalW2 (available on the website of European Bioinformatics Institute: http://www.ebi.ac.uk/Tools/msa/clustalw2/, see also, Higgins D.G. et al, Methods in Enzymology, 266:383-402 (1996); Larkin M.A. et al, Bioinformatics (Oxford, England), 23(21): 2947-8 (2007)), and TCoffee (available on the website of Swiss Institute of Bioinformatics, see also, Poirot O. et al, Nucleic Acids Res., 31(13): 3503-6 (2003); Notredame C. et al, J. Mol. Boil., 302(1): 205-17 (2000)). Those skilled in the art may use the default parameters provided by the tool, or may customize the parameters as appropriate for the alignment, such as for example, by selecting a suitable algorithm.

The polypeptides provided herein further encompass their functional equivalents. A "functional equivalent" as used herein, refers to a polypeptide which possesses functional or structural characteristics that are substantially similar to all or part of a parent polypeptide. The polypeptides provided herein encompass their functional equivalents that can exert a substantially similar function, i.e. reprograming a somatic cell into a stem cell or changing a cell state from one to another. A functional equivalent of the polypeptides provided herein (i.e. parent polypeptides) may be a fragment, mutant, derivative, variant, or analog of a parent polypeptide, and may contain chemical or biological modifications. The functional equivalent may have one or more amino acid substitutions, additions, deletions, insertions, truncations, modifications (e.g. phosphorylation, glycosylation, labeling, etc.), or any combination thereof, of the parent polypeptide. The functional equivalent may include naturally occurring variants of the parent polypeptide and artificial polypeptide sequences such as those obtained by recombinant methods or chemical synthesis. The functional equivalent may contain non-naturally occurring amino acid residues.

The transducible material further comprises a transduction domain. A transduction domain is a motif that is capable of facilitating the entry of the transducible material into a biological sample (e.g., a cell). The transducible domain is associated with the effector domain covalently, noncovalently or via a linker. In certain embodiments of the invention, the transduction domain is covalently linked to the effector domain through a linker. In certain embodiments of the invention, the linker is a glycine-rich linker that comprises one or more glycine residues (e.g. esggggspg (SEQ ID NO: 55)).

Examples of a transduction domain according to the disclosure include, without limitation, polymers such as cationic lipid polymers and nanoparticles, protein transduction domains (PTD), cell penetrating peptides (CPP1), cell permeating peptides (CPP2), activatable cell penetrating peptides or conjugates (ACPP), cell-targeting peptides (CTP), and supercharged proteins.

CPP1, CPP2, PTD and super charged proteins are peptides known to facilitate delivery of a molecular cargo associated thereof into cells. The association between a CPP1, CPP2 PTD or super charged proteins and the molecular cargo can be through covalent bond or non-covalent interactions. The molecular cargo can be small chemical molecules, peptides, protein, fragment of DNA, RNA such as siRNA and miRNA, or nanosize particles. For example, CPP1 and PTD include 5 to 20 amino acid peptide motifs that are capable of penetrating cells independent of surface transporters and of cell cycle phase. CPP1 and PTD can also be capable of penetrating through blood-brain barriers. CPP1 and PTD can deliver proteins and peptides *in vitro* and *in vivo* with uniform distribution throughout the organism after parenteral administration. Cationic PTDs can act as nuclear localization signals and carry an associated molecular cargo to cell nuclei. Examples of protein transduction domains include, without limitation, TAT (e.g. YGRKKRRQRRR, SEQ ID NO: 34), poly-arginine (e.g. poly-arginine having 7-11 arginine residues such as RRRRRRR, RRRRRRRR, RRRRRRRRR, RRRRRRRRRR (SEQ ID NO: 35)and RRRRRRRRRRR (SEQ ID NO: 36)), Penetratin (Antennapedia, e.g. RQIKIWFQNRRMKWKK (SEQ ID NO: 38)), VP22 (e.g. DAATATRGRSAASRPTQRPRAPARSASRPRRPVQ (SEQ ID NO: 39)), Transportan (e.g. GWTLNSAGYLLGKINLKALAALAKKIL (SEQ ID NO: 40)), MAP (e.g. KLALKLALKALKAALKLA (SEQ ID NO: 41)), MTS (e.g. AAVALLPAVLLALLP (SEQ ID NO: 42)), PEP-1 (e.g. KETWWETWWTEWSQPKKKRKV (SEQ ID NO: 43)), Arg/Trp analogue (e.g. RRWRRWWRRWWRRW (SEQ ID NO: 44)), polyguanidine peptoids (e.g. polyguanidine peptoids with a 6-methylene spacer between backbone and guanidine group such as N-arg 5, 7 or 9 peptoids), inherent protein transduction domain, (e.g. RHIKIWFQNRRMKWKK (SEQ ID NO: 56), KPKRRGPKKKKMTKARLERFKLRRMKANARERNR (SEQ ID NO: 57), HIV-1 Rev (e.g. TRQARRNRRRRWRERQR (SEQ ID NO: 60)), Flock house virus coat peptide (e.g. RRRRNRTRRNRRRVR (SEQ ID NO: 61)), DNA-binding peptides such as c-Fos (e.g. KRRIRRERNKMAAAKSRNRRRELTDT (SEQ ID NO: 62)), c-Jun (e.g. RIKAERKRMRNRIAASKSRKRKLERIAR (SEQ ID NO: 63)), yeast GCN4 (e.g. KRARNTEAARRSRARKLQRMKQ (SEQ ID NO: 64)), Fusogenic HA2 peptide (e.g. GLFGAIAGFIENGWEGMIDG (SEQ ID NO: 65), GDIMGEWGNEIFGAIAGFLG (SEQ ID NO: 66)).

A supercharged protein can be a peptide or protein modified to have an increased or decreased overall net charge (either positive or negative net charge) relative to the unmodified form of the protein. In certain embodiments of the invention, superpositively charged protein, which has an overall net positive charge, can be used as the transduction domain. Examples of the supercharged proteins according to the invention include, without limitation, supercharged GFP (e.g. ASKGERLFRGKVPILVELKGDVNGHKFSVRGKGKGDATRGKLTLKFICTTGKLP VPWPTLVTTLTYGVQCFSRYPKHMKRHDFFKSAMPKGYVQERTISFKKDGKYKTRAEVKFEGRTLVNRIKLKGRDFKEKGNILGHKLRYNFNSHKVYITADKRKNGIKAKFKIRHNVKDGSVQLADHYQQNTPIGRGPVLLPRNHYLSTRSKLSKDPKEKRDHMVLLEFVTAAGIKHGRDERYK (SEQ ID NO: 67)), and, according to the disclosure, other supercharged proteins disclosed in U.S. patent application US20110112040. Naturally occurring supercharged proteins can also be used according to the disclosure, such as HBEGF (Accession No.: Q99075), N-DEK (Accession No.: P35659), C-jun (Accession No.: P05412), and HGF (Accession No.: P14210), and other naturally supercharged proteins as disclosed in U.S. patent application US20110112040.

Cell-targeting peptides are proteins or peptides that bind to cell-surface receptors and enter cells through endocytosis. In certain embodiments of the disclosure, a cell-target peptide targets specific tissues or cell types, for example, GnRH peptides (e.g. SEQ ID NO: 58) target biological samples that express GnRH receptors (e.g. solid tumors and hormone-responsive cancer cell lines). More examples of cell-targeting peptides and the specific biological samples targeted according to the disclosure are listed in Table 2.

**Table 2. Examples of cell-targeting peptides and the specific biological samples targeted.**

| **Sequence ID No.** | **Peptide sequence** | **Length** | **Targeted tissue or cells and the cellular targets** |
|---|---|---|---|
| SEQ ID NO: 45 | TSPLNIHNGQKL | 12 | Human head and neck solid tumors |
| SEQ ID NO: 46 | CGKRK | 5 | Tumor neovasculature, Heparan sulfate |
| SEQ ID NO: 47 | CGNKRTRGC | 7 | breast carcinoma |
| SEQ ID NO: 48 | SMSIARL | 7 | Prostate vasculature |
| SEQ ID NO: 49 | FQHPSFI | 7 | hepatocellular carcinoma cell line |
| | RGD | 3 | Integrin receptor', αVβ3 |
| | NGR | 3 | Tumor neovasculature, Amino-peptidase N |
| SEQ ID NO: 50 | VHSPNKK | 7 | Endothelial VCAM-1 expressing cells; VCAM-1 |
| SEQ ID NO: 51 | RRPYIL | 6 | Adenocarcinoma cells; Neurotensin receptor |
| SEQ ID NO: 52 | EDYELMDLLAYL | 12 | Various carcinoma |
| SEQ ID NO: 53 | LTVSPWY | 7 | breast carcinoma; erbB2 |
| SEQ ID NO: 54 | ATWLPPR | 7 | Tumor neovasculature; VEGF receptor |

An activatable cell penetrating peptide or conjugate (ACPP) comprises a cationic CPP1, CPP2, PTD or super charged proteins and a netutralizing anionic counterpart. In certain embodiments of the disclosure, the cationic CPP1, CPP2, PTD or super charged proteins and the anionic counterpart are associated via noncovalent interactions (e.g. charge-charge interaction) and/or covalent cleavable linker (e.g. matrix metaloprotease (MMP) cleavable sequence). Transduction of an ACPP into cells are inhibited until the noncovalent interactions are disrupted and/or the cleavable linker is cleaved. For example, without being bound to any theory, the anionic counterparts comprise one or more pH sensitive groups such as sulfonamide groups, which are protonated at pH 7.4 (the pH in the blood stream) and become neutral at slightly acidic pH (e.g. pH 6.8). Therefore, charge-charge interactions between cationic CPP1, CPP2, PTD or super charged proteins and the anionic counterpart can be interrupted in slightly acidic microenvironment (e.g. in or around tumor or cancer). MMP concentration in blood stream is lower than that in a microenvironment around tumor or cancer. Therefore, MMP cleavable sequence, which is not cleaved in the bloodstream, is cleaved in environment around tumor or cancer. The cationic CPP1, CPP2, PTD or super charged proteins is no longer neutralized by the anionic counterpart, and therefore is exposed to facilitate the translocation into cells (e.g. tumor or cancer cells). In certain embodiments of the disclosure, the CPP1, CPP2 or PTD is TAT. In certain embodiments of the disclosure, the anionic counterparts comprise pH-sensitive polymer (e.g. di-block copolymer) comprising pH-sensitive groups (e.g. sulfonamide groups).

For another example, an activatable cell penetrating conjugate comprises a conventional hydrophobic core made of a polymer into which an effective domain is incorporated, a peripheral hydrophilic layer composed of poly-ethylene glycol and one or more cationic CPP1s, CPP2s, PTDs or super charged proteins, and one or more anionic counterpart that neutralize the cationic CPP12, CPP2s, PTDs or super charged proteins through charge-charge interactions. Such charge-charge interactions are expected to shield the cationic charges during delivery until the transduction material reaches a slightly acidic microenvironment (e.g. tumor or cancer), which triggers protonation of the anionic counterparts and disrupts the charge-charge association. Subsequently the cationic CPP1s, CPP2s, PTDs or super charged proteins, previously quenched by the anionic counterpart, are now capable of facilitating delivery of the effector domain into the surrounding cells (e.g. tumor or cancer cells).

In certain embodiments of the disclosure, a selective transducible material comprises a transduction domain selected from the group consisting of cell-targeting peptides and activatable cell penetrating peptides and activatable cell penetrating conjugates.

According to the invention, a transduction domain is associated to an effector domain via covalent bond, non-covalent interactions or through a linker. Thus a transducible material can be made by obtaining the transduction domain and the effector domain separately and associate together through a covalent bond or non-covalent interactions (e.g., repulsive interactions, dipole interactions, hydrogen bonding interactions, dispersive interactions, charge-charge interactions, solvent, counter ion and entropic effects, and water and hydrophobic effects). In certain embodiments of the invention, the transduction material is prepared by mixing the effector domain and transducible domain. Alternatively, a transducible material can be produced by isolating the material from natural resources or recombinantly. In the case when both domains are peptides or polypeptides, the effector domain can be linked to the N-terminus or C-terminus of the transduction domain and the transducible polypeptide can be made synthetically through chemical synthesis or recombinantly through recombinant technology.

In certain embodiments of the disclosure, a transducible material comprises an effector domain that is inherently transducible, and a transduction domain associated with the effector domain via covalent or non-covalent interactions.

In certain embodiments of the invention, a transducible material further comprises one or more motifs that do not interrupt the function of the effector domain or the transduction domain. In certain embodiments of the invention, these motifs are linked covalently, non-covalently or through a linker to the effector domain and/or the transduction domain. In certain embodiments of the invention, these motifs facilitate the preparation and/or purification of the transducible material. One example of such motif is a polyhistidine-tag to facilitate protein purification in preparation of the transducible material. In certain embodiments of the invention, the polyhistidine-tag comprises at least six histidine residues (e.g. MGSSHHHHHHSSGLVPRGSH ("His6," SEQ ID NO: 59)).

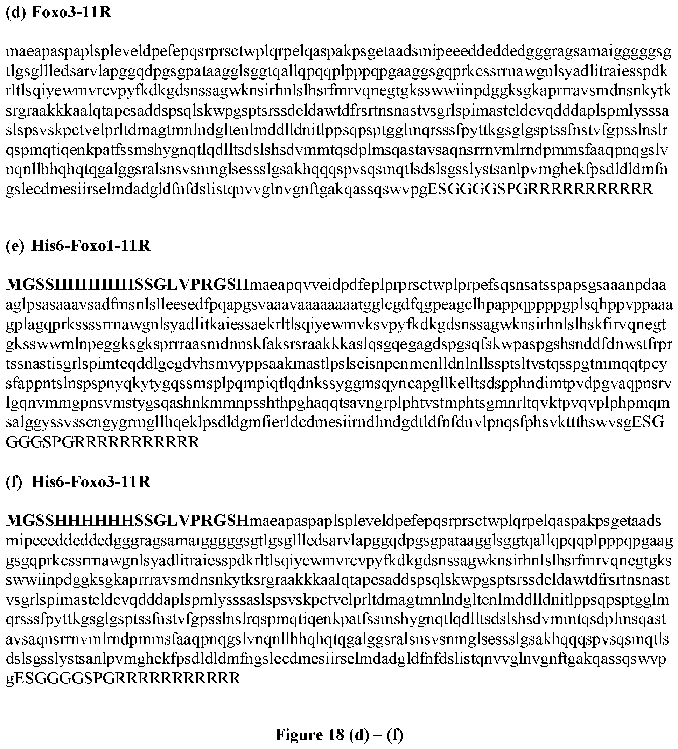

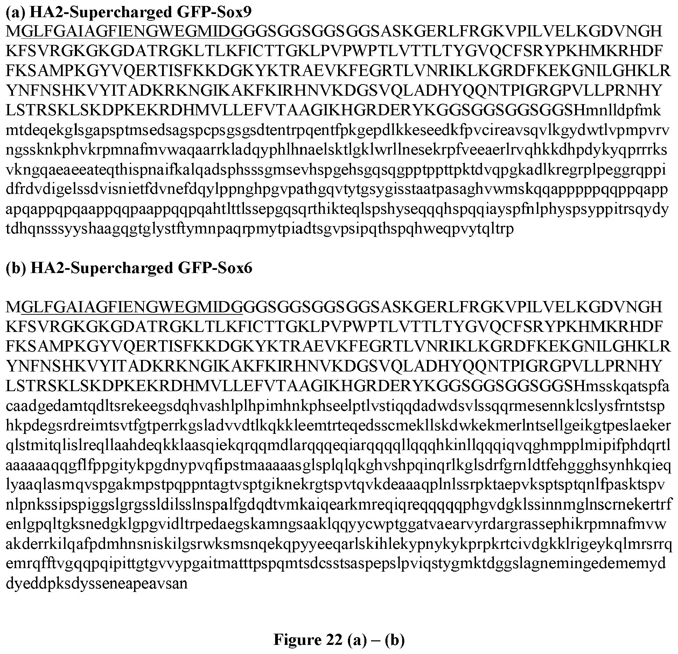

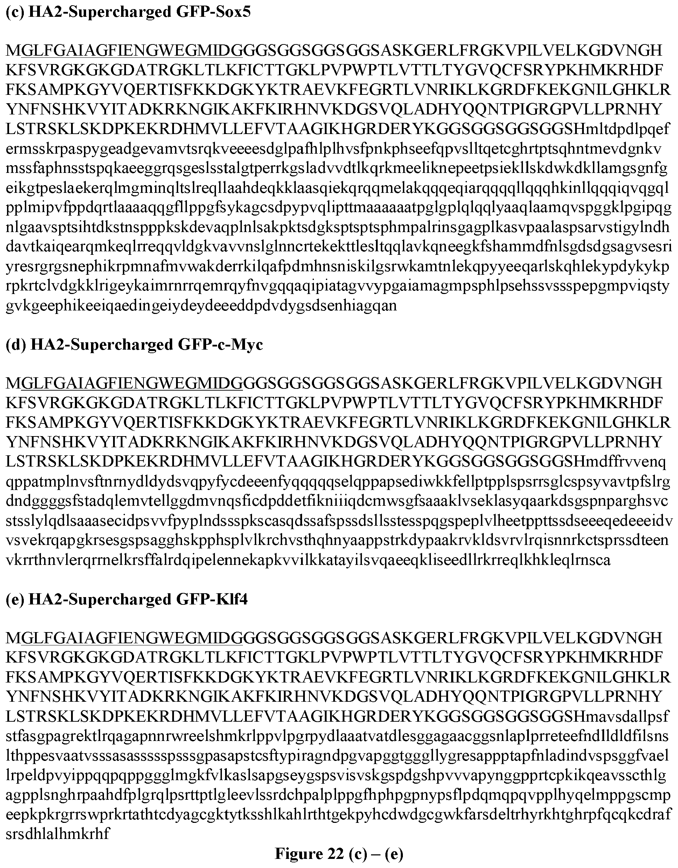

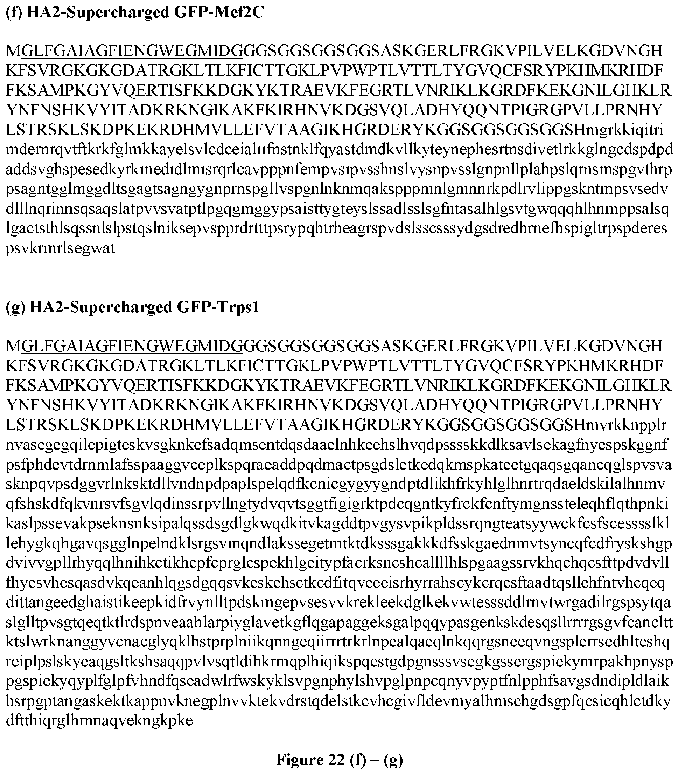

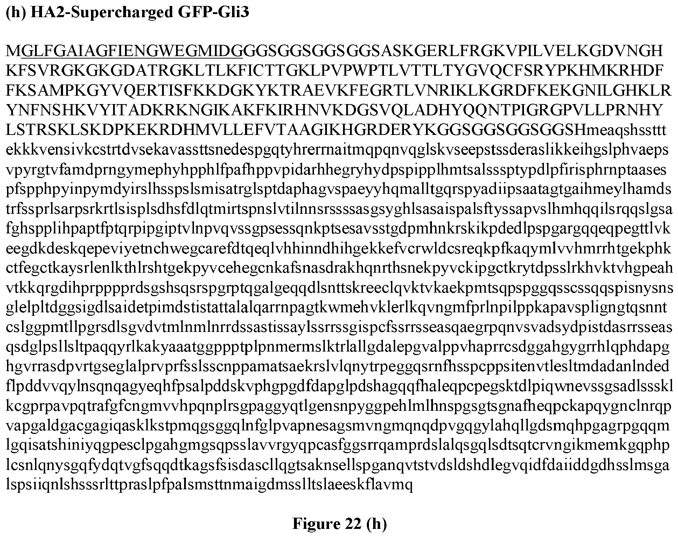

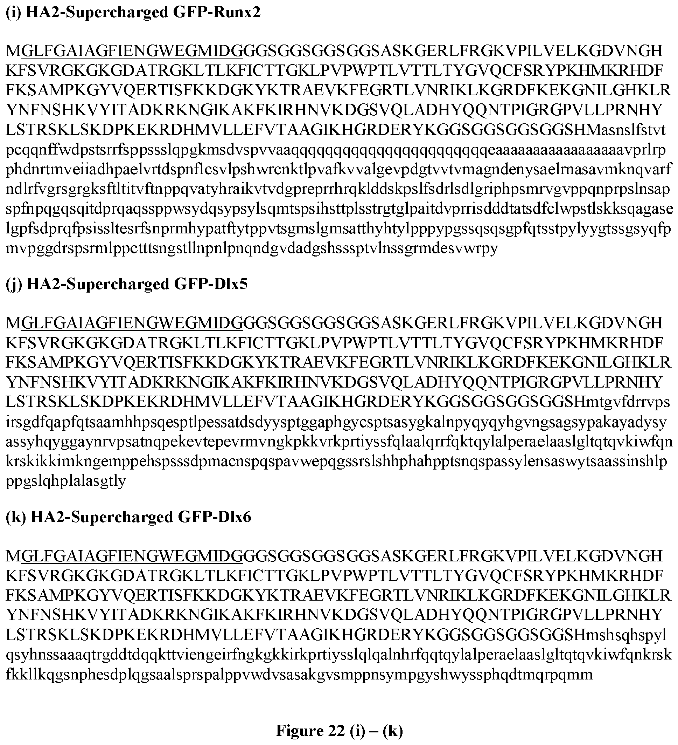

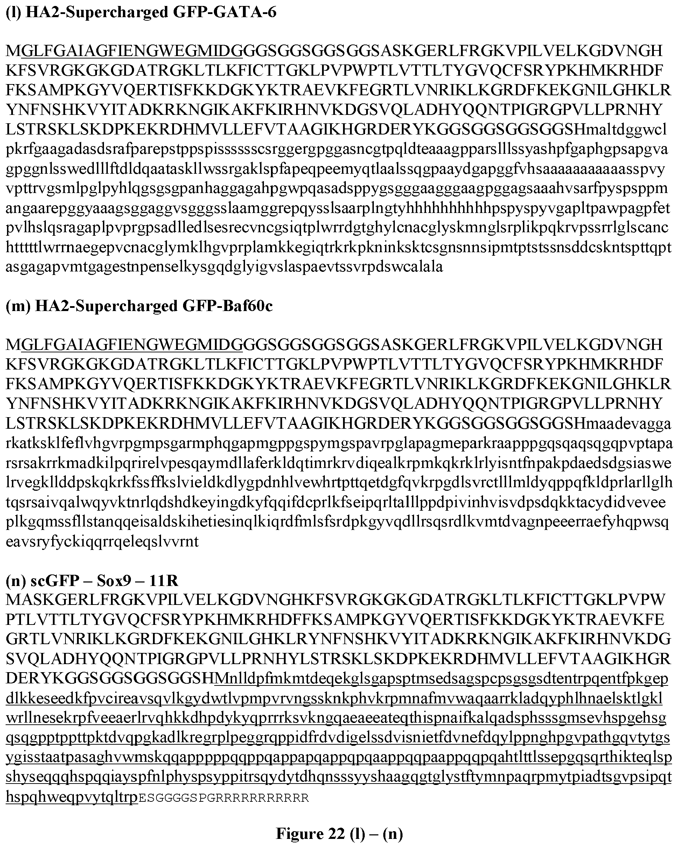

In certain embodiments of the disclosure, a transducible material includes, for example, Oct4-11R (SEQ ID NO: 12), Sox2-11R (SEQ ID NO: 13), K1f4-11R (SEQ ID NO: 14), Lin28-11R (SEQ ID NO: 16), Nanog-11R (SEQ ID NO: 17), cMyc-11R (SEQ ID NO: 15), Ngn3-11R (SEQ ID NO: 19), PDX1-11R (SEQ ID NO: 20), MafA-11R (SEQ ID NO: 21), NeuroD-11R (SEQ ID NO: 18), Foxp3-11R (SEQ ID NO: 22), Foxo1-11R (e.g. Figure 18c), Foxo3-1 1R (e.g. Figure 18d), Sox9-11R, Sox6-11R, Sox5-11R, c-Myc-11R, K1f4-11R, Mef2C-11R, Trps1-11R, Gli3-11R, Runx2-11R, Dlx5-11R, Dlx6-11R, GATA-6-11R and Baf60c-11R, wherein 11R (SEQ ID NO: 37) stands for a polyarginine sequence of 11 arginine residues linking to a linker through which the polyarginie sequence is covalently linked to the effector domain. In certain embodiments of the invention, the "11R" is covalently linked to the C terminal of the effector domain. In certain embodiments of the disclosure, a transducible material includes, for example, His6-Oct4-11R (SEQ ID NO: 23), His6-Sox2-11R (SEQ ID NO: 24), His6-Klf4-11R (SEQ ID NO: 25), His6-Lin28-11R (SEQ ID NO: 27), His6-Nanog-11R (SEQ ID NO: 28), His6-cMyc-11R (SEQ ID NO: 26), His6-Ngn3-11R (SEQ ID NO: 30), His6-PDX1-11R (SEQ ID NO: 31), His6-MafA-11R (SEQ ID NO: 32), His6-NeuroD-11R (SEQ ID NO: 29), His6-Foxp3-11R (SEQ ID NO: 33), His6-Foxo1-11R (e.g. Figure 18e), His6-Foxo3-11R (e.g. Figure 18f), His6-Sox9-11R, His6-Sox6-11R, His6-Sox5-11R, His6-c-Myc-11R, His6-Klf4-11R, His6-Mef2C-11R, His6-Trps1-11R, His6-Gli3-11R, His6-Runx2-11R, His6-Dlx5-11R, His6-Dlx6-11R, His6-GATA-6-11R, His6-Baf60c-11R, HA2-Supercharged GFP-Sox9, HA2-Supercharged GFP-Sox6, HA2-Supercharged GFP-Sox5, HA2-Supercharged GFP-c-Myc, HA2-Supercharged GFP-Klf4, HA2-Supercharged GFP-Mef2C, HA2-Supercharged GFP-Trps1, HA2-Supercharged GFP-Gli3, HA2-Supercharged GFP-Runx2, HA2-Supercharged GFP-Dlx5, HA2-Supercharged GFP-Dlx6, HA2-Supercharged GFP-GATA-6, HA2-Supercharged GFP-Baf60c, scGFP-Sox9-11R, scGFP-Sox6-11R, scGFP-Sox5-11R, scGFP-c-Myc-11R, scGFP-Klf4-11R, scGFP-Mef2C-11R, scGFP-Trps1-11R, scGFP-Gli3-11R, scGFP-Runx2-11R, scGFP-Dlx5-11R, scGFP-Dlx6-11R, scGFP-GATA-6-11R, and scGFP-Baf60c-11R. In certain embodiments of the disclosure, the "His6" is covalently linked to the N terminal of the effector domain. In certain embodiments of the invention, the N-terminal of the transcription factor is linked to scGFP via a first linker (e.g., GGSGGSGGSGGSH), and the C-terminal of the transcription factor is covalently linked to 11R with or without a second linker. A schematic presentation of a scGFP fused transcription material is shown in Figure 1 (c), and amino acid sequence of an exemplary scGFP fused transcription material (i.e. scGFP-Sox9-11R) is provided in Figure 22 (n), in which the sequence of the transcription factor Sox9 is underlined. By replacing the underlined Sox9 sequence with another transcription factor sequence, one can obtain the sequences of the scGFP fused transcription materials for the transcription factors, e.g. those listed in Table 1. Exemplary sequences of transducible materials of the disclosure are shown in Table 3.

**Table 3 Exemplary sequences of transducible materials**

| Effector Domain | Effector domain-11R (Exemplary sequence) | His6 - Effector domain-11R (Exemplary sequence) | HA2-Supercharged GFP-Effector domain (Exemplary sequence) |
|---|---|---|---|
| Sox9 | Sox9-11R (Figure 20 (a)) | His6-Sox9 -11R (Figure 21 (a)) | HA2-Supercharged GFP-Sox9 (Figure 22 (a)) scGFP-Sox9-11R (Figure 22 (n), SEQ ID NO: 68) |
| Sox6 | Sox6-11R (Figure 20 (b)) | His6 - Sox6-11R (Figure 21 (b)) | HA2-Supercharged GFP-Sox6 (Figure 22 (b)) |
| Sox5 | Sox5-11R (Figure 20 (c)) | His6 - Sox5-11R (Figure 21 (c)) | HA2-Supercharged GFP-Sox5 (Figure 22 (c)) |
| c-Myc | c-Myc -11R (Figure 20 (d)) | His6 - c-Myc -11R (Figure 21 (d)) | HA2-Supercharged GFP-c-Myc (Figure 22 (d)) |
| Klf4 | Klf4-11R (Figure 20 (e)) | His6 - K1f4-11R (Figure 21 (e)) | HA2-Supercharged GFP-Klf4 (Figure 22 (e)) |
| Mef2C | Mef2C -11R (Figure 20 (f)) | His6 - Mef2C -11R (Figure 21 (f)) | HA2-Supercharged GFP-Mef2C (Figure 22 (f)) |
| Trps1 | Trps1-11R (Figure 20 (g)) | His6 - Trps1-11R (Figure 21 (g)) | HA2-Supercharged GFP-Trps1 (Figure 22 (g)) |
| Gli3 | GH3-11R (Figure 20 (h)) | His6 - Gli3-11R (Figure 21 (h)) | HA2-Supercharged GFP-Gli3 (Figure 22 (h)) |
| Runx2 | Runx2-11R (Figure 20 (i)) | His6 - Runx2-11R (Figure 21 (i)) | HA2-Supercharged GFP-Runx2 (Figure 22 (i)) |
| Dlx5 | Dlx5-11R (Figure 20 (j)) | His6 - Dlx5-11R (Figure 21 (j)) | HA2-Supercharged GFP-Dlx5 (Figure 22 (j)) |
| D1x6 | Dlx6-11R (Figure 20 (k)) | His6 - Dlx6-11R (Figure 21 (k)) | HA2-Supercharged GFP-D1x6 (Figure 22 (k)) |
| GATA-6 | GATA-6-11R (Figure 20 (l)) | His6 - GATA-6-11R (Figure 21 (l)) | HA2-Supercharged GFP-GATA-6 (Figure 22 (l)) |
| Baf60c | Baf60c -11R (Figure 20 (m)) | His6 - Baf60c -11R (Figure 21 (m)) | HA2-Supercharged GFP-Baf60c (Figure 22 (m)) |

In certain embodiments of the invention, a transducible material can be combined with one or more adjuvants such as small molecule epigenetic agents. Suitable epigenetic agents include, without limitation, histone deacetylase inhibitor and DNA methylation inhibitor. Examples of suitable adjuvants include, without limitation, trichostatin A, which is a histone deacetylase inhibitor and DNA methylation inhibitor, valproic acid, which is a histone deacetylase inhibitor and DNA methylation inhibitor, aza-2'-deoxycytidine, which is a DNA methylation inhibitor, and suberoylanilide hydroxamic acid, which is a histone deacetylase inhibitor.

Another aspect of the present disclosure relates to a composition comprising a biological sample and at least one transducible material, wherein the transducible material has transduced into the biological sample. For example, the composition includes a transducible material comprising Foxp3, Foxo1, or Foxo3, or any combination thereof (e.g. the transducible material is Foxp3, Foxp3-11R, His6-Foxp3-11R, Foxo1, Foxo1-11R, His6-Foxo1-11R, Foxo3, Foxo3-11R, or His6-Foxo3-11R) and a T cell wherein the transducible material has transduced into the T cell; a composition includes a piPS cell and one or more transducible materials comprising a polypeptide selected from the group consisting of Oct4, Klf4, Sox2 and cMyc, and any combination thereof (e.g. the transducible material is Oct4, Klf4, Sox2, cMyc, Oct4-11R, K1f4-11R, Sox2-11R, cMyc-11R, His6-Oct4-11R, His6-Klf4-11R, His6-Sox2-11R or His6-cMyc-11R); a composition including a liver or pancreatic exocrine cell and one or more transducible materials comprising a polypeptide selected from the group consisting of Ngn3, PDX1, MafA, NeuroD, and any combination thereof (e.g. the transducible material is Ngn3, PDX1, MafA, NeuroD, Ngn3-11R, PDX1-11R, MafA-11R, NeuroD-11R, His6-Ngn3-11R, His6-PDX1-11R or His6-MafA-11R, His6-NeuroD-11R) wherein the transducible materials have transduced into the liver or pancreatic exocrine cell; and a composition including a starting or substrate cell (e.g. embryonic stem cells (ESCs), induced pluripotent stem cells (iPSCs), mesenchymal stem cells (MSCs), fibroblasts, hematopoietic stem cells, endothelian stem cells, adipocytes, chondrocytes, osteoblasts, osteoclasts and endothelial cells) and one or more transducible materials comprising a polypeptide selected from the group consisting of Sox9, Sox6, Sox5, c-Myc, Klf4, Mef2C, Trps1, Gli3, Runx2, Dlx5, Dlx6, GATA-6 and Baf60c, and any combination thereof (e.g. the transducible material is Sox9-11R, Sox6-11R, Sox5-11R, c-Myc-11R, K1f4-11R, Mef2C-11R, Trps1-11R, Gli3-11R, Runx2-11R, D1x5-11R, Dlx6-11R, GATA-6-11R, Baf60c-11R, His6-Sox9-11R, His6-Sox6-11R, His6-Sox5-11R, His6-c-Myc-11R, His6-Klf4-11R, His6-Mef2C-11R, His6-Trpsl-11R, His6-Gli3-11R, His6-Runx2-11R, His6-Dlx5-11R, His6-D1x6-11R, His6-GATA-6-11R, His6-Baf60c-11R, HA2-Supercharged GFP-Sox9, HA2-Supercharged GFP-Sox6, HA2-Supercharged GFP-Sox5, HA2-Supercharged GFP-c-Myc, HA2-Supercharged GFP-Klf4, HA2-Supercharged GFP-Mef2C, HA2-Supercharged GFP-Trps1, HA2-Supercharged GFP-Gli3, HA2-Supercharged GFP-Runx2, HA2-Supercharged GFP-Dlx5, HA2-Supercharged GFP-Dlx6, HA2-Supercharged GFP-GATA-6, HA2-Supercharged GFP-Baf60c, scGFP-Sox9-11R, scGFP-Sox6-11R, scGFP-Sox5-11R, scGFP-c-Myc-11R, scGFP-Klf4-11R, scGFP-Mef2C-11R, scGFP-Trps1-11R, scGFP-Gli3-11R, scGFP-Runx2-11R, scGFP-Dlx5-11R, scGFP-D1x6-11R, scGFP-GATA-6-11R, scGFP-Baf60c-11R or any combination thereof) wherein the transducible materials have transduced into the starting cell.

Another aspect of the present disclosure relates to a method of reprogramming a biological sample by exposing the biological sample to a composition comprising a transducible material of the present disclosure. In certain embodiments of the disclosure, the method preferably reprograms a specific type of biological sample (e.g. cancer or tumor cells) or biological samples in or around a specific microenvironment within a biological organism (e.g. microenvironment around cancer or tumor) than other biological samples by exposing biological samples to a composition comprising a selective transducible material.

In one embodiment of the disclosure, a biological sample includes a cell, a cluster of cells, a tissue, an organ, a biological body from a biological organism. The biological sample can be normal, healthy sample or abnormal, diseased sample (e.g., cancer or tumor).

A biological organism includes, for example, a microorganism (e.g., bacteria), a fungus, a plant and an animal (e.g., a human).

An organ from an animal biological organism (e.g., human) includes, for example, a circulatory organ (e.g., heart, blood and blood vessels), a digestive organ (e.g., salivary glands, esophagus, stomach, liver, gallbladder, pancreas, intestines, rectum and anus), an endocrine organ (e.g., endocrine glands such as the hypothalamus, pituitary or pituitary gland, pineal body or pineal gland, thyroid, parathyroids and adrenals, i.e., adrenal glands), an integumentary organ (e.g., skin, hair and nails), a lymphatic organ (e.g., lymph nodes and vessels, tonsils, adenoids, thymus and spleen), a muscular organ (e.g., muscles), a nervous organ (e.g., brain, spinal cord, peripheral nerves and nerves), a reproductive organ (e.g., ovaries, fallopian tubes, uterus, vagina, mammary glands, testes, vas deferens, seminal vesicles, prostate and penis), a respiratory organ (e.g., the pharynx, larynx, trachea, bronchi, lungs and diaphragm), a skeletal organ (e.g., bones, cartilage, ligaments and tendons), a urinary system (e.g., kidneys, ureters, bladder and urethra). An organ can be normal or healthy, and alternatively, abnormal or unhealthy (e.g., cancerous).

An organ from a plant biological organism includes, for example, root, stem, leaf, flower, seed and fruit.

A tissue from a biological sample (e.g. an animal) includes a connective tissue, a muscle tissue, a nervous tissue, and an epithelial tissue. A tissue can be normal or healthy, and alternatively, abnormal or unhealthy (e.g., cancerous). A tissue from a biological sample (e.g. a plant) includes an epidermis, a vascular tissue and a ground tissue.

A cell can be prokaryotic or eukaryotic. A prokaryotic cell includes, for example, bacteria. A eukaryotic cell includes, for example, a fungus, a plant cell, and an animal cell. The types of an animal cell (e.g., a mammalian cell or a human cell) includes, for example, a cell from circulatory/immune system or organ (e.g., a B cell, a T cell (cytotoxic T cell, natural killer T cell, regulatory T cell, T helper cell), a natural killer cell, a granulocyte (e.g., basophil granulocyte, an eosinophil granulocyte, a neutrophil granulocyte and a hypersegmented neutrophil), a monocyte or macrophage, a red blood cell (e.g., reticulocyte), a mast cell, a thrombocyte or megakaryocyte, and a dendritic cell); a cell from an endocrine system or organ (e.g., a thyroid cell (e.g., thyroid epithelial cell, parafollicular cell), a parathyroid cell (e.g., parathyroid chief cell, oxyphil cell), an adrenal cell (e.g., chromaffin cell), and a pineal cell (e.g., pinealocyte)); a cell from a nervous system or organ (e.g., a glioblast (e.g., astrocyte and oligodendrocyte), a microglia, a magnocellular neurosecretory cell, a stellate cell, a boettcher cell, and a pituitary cell (e.g., gonadotrope, corticotrope, thyrotrope, somatotrope, and lactotroph)); a cell from a respiratory system or organ (e.g., a pneumocyte (a type I pneumocyte and a type II pneumocyte), a clara cell, a goblet cell, an alveolar macrophage); a cell from circular system or organ (e.g., myocardiocyte and pericyte); a cell from digestive system or organ (e.g., a gastric chief cell, a parietal cell, a goblet cell, a paneth cell, a G cell, a D cell, an ECL cell, an I cell, a K cell, an S cell, an enteroendocrine cell, an enterochromaffin cell, an APUD cell, a liver cell (e.g., a hepatocyte and Kupffer cell)); a cell from integumentary system or organ (e.g., a bone cell (e.g., an osteoblast, an osteocyte, and an osteoclast), a teeth cell (e.g., a cementoblast, and an ameloblast), a cartilage cell (e.g., a chondroblast and a chondrocyte), a skin/hair cell (e.g., a trichocyte, a keratinocyte, and a melanocyte (Nevus cell)), a muscle cell (e.g., myocyte), an adipocyte, a fibroblast, and a tendon cell), a cell from urinary system or organ (e.g., a podocyte, a juxtaglomerular cell, an intraglomerular mesangial cell, an extraglomerular mesangial cell, a kidney proximal tubule brush border cell, and a macula densa cell), and a cell from reproductive system or organ (e.g., a spermatozoon, a sertoli cell, a leydig cell, an ovum, an oocyte). A cell can be normal, healthy cell; or a diseased or unhealthy cell (e.g., a cancer cell).

A cell further includes a mammalian stem cell which include an embryonic stem cell, a fetal stem cell, an induced pluripotent stem cell, and an adult stem cell. A stem cell is a cell that is capable of undergoing cycles of cell division while maintaining an undifferentiated state and differentiating into specialized cell types. A stem cell can be an omnipotent stem cell, a pluripotent stem cell, a multipotent stem cell, an oligopotent stem cell and an unipotent stem cell (See, Hans R. Schöler (2007). "The Potential of Stem Cells: An Inventory" in Nikolaus Knoepffler, Dagmar Schipanski, and Stefan Lorenz Sorgner. Humanbiotechnology as Social Challenge. Ashgate Publishing, Ltd. pp. 28), any of which may be induced from a somatic cell. A stem cell may also include a cancer stem cell.

In certain embodiments of the disclosure, the cell is embryonic stem cells (ESCs), induced pluripotent stem cells (iPSCs), MSCs, fibroblasts, hematopoietic stem cells, endothelian stem cells, adipocytes, chondrocytes, osteoblasts, osteoclasts or endothelial cells.

In another embodiment of the disclosure, "reprogramming a biological sample" used herein is exchangeable with or refers to modulating, altering, or changing the biological activities of the biological sample (e.g., cell) or modulating, altering, or changing the state or status of the biological sample from one to another. For example, by exposing a biological sample (e.g., a cell) to a transducible material, the biological activities of the cell (e.g., cell growth, cell division, cell metabolism, cell cycle, cell signaling, DNA replication, transcription, RNA splicing, protein synthesis, post-translation modification) are modulated or altered so as to lead to cell proliferation, differentiation (e.g., from progenitor cells to terminally differentiated cells), dedifferentiation (e.g., from terminally differentiated cells to pluripotent stem cells), transdifferentiation (e.g., from one type of terminally differentiated cells to another type of terminally differentiated cells), retrodifferentiation (e.g., from terminally differentiated cells to progenitor cells), transdertermination (e.g., from one type of progenitor cells to a type of terminally differentiated cells that are usually derived from another type of progenitor cells under natural conditions), apoptosis (e.g., cell death of cells or cancer cells), morphogenesis, and changes in the cell fate. For another example, the state of a biological sample can be altered or changed from abnormal or diseased state to normal or healthy state (e.g., from cancer cells to noncancer cells); from one cell type to another cell type (e.g., from undifferentiated stem cells to differentiated stem cells or specialized cells), from differentiated or specialized cells to undifferentiated cells or stem cells (e.g., an omnipotent stem cell, a pluripotent stem cell, a multipotent stem cell, an oligopotent stem cell and an unipotent stem cell)(e.g., from fibroblast cells to induced pluripotent stem cells (iPSCs)), from somatic cells to stem cells or induced stem cells, from one state of stem cells to another state of stem cells (e.g., from ominipotent stem cells to pluripotent stem cells), from one type of differentiated cells to another type of differentiated cells (e.g., T-cells to regulatory T cells, pancreatic exocrine cells to insulin-producing beta cells), or from starting cells (e.g. ESCs, iPSCs, MSCs, fibroblasts, hematopoietic stem cells, endothelian stem cells, adipocytes, chondrocytes, osteoblasts, osteoclasts or endothelial cells) to chondrogenic cells.

In another embodiment of the disclosure, a biological sample is exposed to a transducible material and reprogrammed. The biological sample can be exposed *in vitro, in vivo* or *ex vivo.* For example, the biological sample is exposed *in vitro* through contacting the sample with the transducible material in an environment outside of a living biological organism (e.g., in a cell culture system or a test tube). The biological sample is exposed *in vivo* through contacting the material with a biological organism containing the sample or introducing (e.g., through administration) the material into the organism. The transducible materials can be administered via any known administration route such as for example parenteral (*e.g.,* subcutaneous, intraperitoneal, intravenous, including intravenous infusion, intramuscular, or intradermal injection) or non-parenteral (*e.g.,* oral, intranasal, intraocular, sublingual, rectal, or topical) route. The biological sample is exposed *ex vivo* when the biological sample (e.g., a cell, a tissue or an organ) is taken outside the biological organism, contacted with the transducible material, and placed back to the same or different biological organisms. Examples of *ex vivo* exposures comprise removing a biological sample from the biological organism, exposing the biological sample to a transducible material, and transplanting the biological sample transduced with the transducible material back to the biological organism.

In certain embodiments of the disclosure, OG2-MEF cells are exposed to a composition comprising protein Oct4-11R, Sox2-11R, K1f4-11R and cMyc-11R and reprogrammed to induced pluripotent stem cells (iPSCs).

In certain embodiments of the disclosure, T cells are exposed to a composition comprising protein Foxp3-11R, His6-Foxp3-11R, Foxo1-11R, His6-Foxo1-11R, His6-Foxo3-11R, Foxo3-11R, and reprogrammed to regulatory T cells (Treg cells).

In certain embodiments of the disclosure liver and/or pancreatic exocrine cells are exposed to a composition comprising one or more proteins selected from the group consisting of Ngn3-11R, PDX1-11R, MafA-11R, NeuroD-11R, His6-Ngn3-11R, His6-PDX1-11R, His6-MafA-11R, and His6-NeuroD-11R and reprogrammed into insulin producing cells (e.g. β cells). In certain embodiments of the disclosure, the composition further comprises one or more adjuvant such as Islet growth factor (e.g. betacellulin). In certain embodiments of the disclosure, the composition comprises His6-Ngn3-11R, His6-PDX1-11R, and His6-MafA-11R. In certain embodiments of the disclosure, the composition comprises His6-Ngn3-11R, His6-PDX1-11R, His6-MafA-11R and betacellulin. Without intending to be bound to a particular mechanism, it is further contemplated that such reprogramming is through transdetermination and/or transdifferentiation.

In certain embodiments of the disclosure, MSCs, ESCs, iPSCs, fibroblasts, hematopoietic stem cells, endothelian stem cells, adipocytes, chondrocytes, osteoblasts, osteoclasts or endothelial cells are exposed to a composition comprising one or more proteins selected from the group consisting of Sox9-11R, Sox6-11R, Sox5-11R, c-Myc-11R, K1f4-11R, Mef2C-11R, Trps1-11R, Gli3-11R, Runx2-11R, D1x5-11R, Dlx6-11R, GATA-6-11R, Baf60c-11R, His6-Sox9-11R, His6-Sox6-11R, His6-Sox5-11R, His6-c-Myc-11R, His6-Klf4-11R, His6-Mef2C-11R, His6-Trpsl-11R, His6-Gli3-11R, His6-Runx2-11R, His6-Dlx5-11R, His6-D1x6-11R, His6-GATA-6-11R, His6-Baf60c-11R, HA2-Supercharged GFP-Sox9, HA2-Supercharged GFP-Sox6, HA2-Supercharged GFP-Sox5, HA2-Supercharged GFP-c-Myc, HA2-Supercharged GFP-Klf4, HA2-Supercharged GFP-Mef2C, HA2-Supercharged GFP-Trps1, HA2-Supercharged GFP-Gli3, HA2-Supercharged GFP-Runx2, HA2-Supercharged GFP-Dlx5, HA2-Supercharged GFP-Dlx6, HA2-Supercharged GFP-GATA-6 and HA2-Supercharged GFP-Baf60c, scGFP-Sox9-11R, scGFP-Soxo-11R, scGFP-Sox5-11R, scGFP-c-Myc-11R, scGFP-Klf4-11R, scGFP-Mef2C-11R, scGFP-Trps1-11R, scGFP-Gli3-11R, scGFP-Runx2-11R, scGFP-Dlx5-11R, scGFP-D1x6-11R, scGFP-GATA-6-11R, and scGFP-Baf60c-11R and reprogrammed into chondrogenic cells. In certain embodiments of the disclosure, the composition further comprises one or more adjuvant such as epigenetic agents (e.g. trichostatin A, valproic acid, aza-2'-deoxycytidine, suberoylanilide hydroxamic acid).

Another aspect of the present disclosure relates to a method of treating, preventing or reducing a disease or condition involving cartilage damage in a biological organism by administering a composition comprising a transducible material of the present disclosure into the organism. In certain embodiments of the disclosure, the composition is a pharmaceutical composition comprising a transducible material. In certain embodiments of the disclosure, the composition comprises a selective transducible material. The treatment, prevention or reduction of a disease or condition is associated with the change or reprogramming of a biological sample (e.g., a cell, a tissue or an organ) in the organism.

The present disclosure also provides use of a transducible material in manufacturing a medicament for treating preventing or reducing a disease or condition involving cartilage damage in a biological organism. In certain embodiments of the disclosure, the transducible material is a selective transducible material. The treatment, prevention or reduction of a disease or condition is associated with the change or reprogramming of a biological sample (e.g., a cell, a tissue or an organ) in the organism. The disease or condition involving cartilage damage of the invention include, without limitations, joint disorder, osteoarthritis, cartilage injury, traumatic rupture or detachment, achondroplasia, costochondritis, spinal disc herniation, relapsing polychonritis, tumor, chondroma, chondrosarcoma, and peomorphic adenoma.

In certain embodiments of the disclosure, the disease or condition treatable by the method or treatable by the medicament include, without limitations, tumor, cancer, metabolic diseases or conditions (e.g. type I and type II diabetes and obesity), inflammatory conditions, cardiac diseases, neurogenerative diseases (e.g. anemia, amyotrophic lateral sclerosis, spinal cord injury, burns, or arthritis), autoimmune diseases or conditions (e.g. acute disseminated encephalomyelitis (ADEM), Addison's disease, alopecia areata, ankylosing spondylitis, antiphospholipid antibody syndrome (APS), anemia (e.g. autoimmune hemolytic anemia and pernicious anaemia), arthritis, psoriatic arthritis, rheumatoid arthritis, diabetes mellitus type 1, autoimmune hepatitis, autoimmune inner ear disease, bullous pemphigoid, coeliac disease, Chagas disease, chronic obstructive pulmonary disease, Crohns disease, dermatomyositis, endometriosis, Goodpasture's syndrome, Graves' disease, Guillain-Barre syndrome (GBS), Hashimoto's disease, hidradenitis suppurativa, Kawasaki disease, IgA nephropathy, idiopathic thrombocytopenic purpura, interstitial cyctitis, lupus erythematosus, mixed connective tissue disease, morphea, multiple sclerosis (MS), myasthenia gravis, narcolepsy, neuromyotonia, pemphigus vulgaris, psoriasis, polymyositis, primary billiary cirrhosis, schizophrenia, scleroderma, Sjogren's syndrome, stiff person syndrome, temporal arteritis ("Giant cell arteritis"), ulcerative colitis, vasculitis, vitiligo, and Wegener's granulomatosis).

For example, it is contemplated that a transducible material, or a medicament manufactured from a transducible material can be administered to a biological organism having a tumor to activate the apoptosis of the tumor cells or make tumor cells more sensitive to chemotherapy, radiotherapy, or cancer drugs.

In certain embodiments of the disclosure, a transducible material, or a medicament manufactured from a transducible material can be administered to a biological organism to enhance or attenuate immune system and thus treat or prevent immune-related diseases or inflammatory diseases. For example, protein Foxp3-11R, His6-Foxp3-11R, Foxo1-11R, His6-Foxo1-11R, His6-Foxo3-1 1R, Foxo3-11R, is transduced to T cells and programs them to Treg cells, which suppress the overactive immune system and thus is a treatment for auto-immune diseases.

In certain embodiments of the invention, a transducible material, or a medicament manufactured from a transducible material can be administered to a biological organism to treat a condition involving cartilage damage such as joint disorder, osteoarthritis, cartilage injury, cartilage injury, traumatic rupture or detachment, achondroplasia, costochondritis, spinal disc herniation, relapsing polychonritis, tumor, chondroma, chondrosarcoma, or peomorphic adenoma.

For example, to treat a condition involving cartilage damage, a polypeptide or a composition comprising such polypeptide is transduced to a suitable cell (e.g. ESCs, iPSCs, MSCs, fibroblasts, hematopoietic stem cells, endothelian stem cells, adipocytes, chondrocytes, osteoblasts, osteoclasts or endothelial cells) and reprograms them to chondrogenic cells, which polypeptide is selected from the group consisting of Sox9-11R, Sox6-11R, Sox5-11R, c-Myc-11R, K1f4-11R, Mef2C-11R, Trps1-11R, Gli3-11R, Runx2-11R, Dlx5-11R, Dlx6-11R, GATA-6-11R, Baf60c-11R, His6-Sox9-11R, His6-Sox6-11R, His6-Sox5-11R, His6-c-Myc-11R, His6-Klf4-11R, His6-Mef2C-11R, His6-Trpsl-11R, His6-Gli3-11R, His6-Runx2-11R, His6-Dlx5-11R, His6-D1x6-11R, His6-GATA-6-11R, His6-Baf60c-11R, HA2-Supercharged GFP-Sox9, HA2-Supercharged GFP-Sox6, HA2-Supercharged GFP-Sox5, HA2-Supercharged GFP-c-Myc, HA2-Supercharged GFP-Klf4, HA2-Supercharged GFP-Mef2C, HA2-Supercharged GFP-Trps1, HA2-Supercharged GFP-Gli3, HA2-Supercharged GFP-Runx2, HA2-Supercharged GFP-Dlx5, HA2-Supercharged GFP-Dlx6, HA2-Supercharged GFP-GATA-6, HA2-Supercharged GFP-Baf60c, scGFP-Sox9-11R, scGFP-Sox6-11R, scGFP-Sox5-11R, scGFP-c-Myc-11R, scGFP-Klf4-11R, scGFP-Mef2C-11R, scGFP-Trps1-11R, scGFP-Gli3-11R, scGFP-Runx2-11R, scGFP-Dlx5-11R, scGFP-D1x6-11R, scGFP-GATA-6-11R, scGFP-Baf60c-11R, and any combination thereof. Without intending to be bound to a particular mechanism, it is further contemplated that such reprogramming is through transdetermination and/or transdifferentiation.

For another example, starting cells or substrate cells (e.g. embryonic stem cells (ESCs), induced pluripotent stem cells (iPSCs), MSCs, fibroblasts, hematopoietic stem cells, endothelian stem cells, adipocytes, chondrocytes, osteoblasts, osteoclasts and endothelial cells) are reprogrammed to chondrogenic cells through differentiation, transdifferentiation, trans-determination, or retro-differentiation.

In certain embodiments of the invention, one or more adjuvant such as epigenetic agents (e.g. trichostatin A, valproic acid, aza-2'-deoxycytidine, and/or suberoylanilide hydroxamic acid) is/are also administered to the biological organism.

Another aspect of the present disclosure relates to a method of reprogramming iPSCs, embryonic stem cells, or other types of stem or progenitor or differentiated cells to chondrogenic cells, which can be developed as cell-based therapies for diseases or conditions involving cartilage damage including joint disorder, osteoarthritis, cartilage injury, traumatic rupture or detachment, achondroplasia, costochondritis, spinal disc herniation, relapsing polychonritis, tumor, chondroma, chondrosarcoma, or peomorphic adenoma. The stem cells, progenitor cells or differentiated cells may be patient-specific or non-patient-specific, repaired to rid of molecular defects or not, before they are exposed to transducible materials for controlled differentiation or reprogramming. The reprogrammed cells may be enriched, purified, or manipulated before transplanted back to patients. In certain embodiments of the disclosure, the reprogrammed cell is chondrogenic cells or their derived cells.

Another aspect of the present disclosure relates to a method of reprogramming iPSCs, embryonic stem cells, or other types of stem or progenitor cells or differentiated cells to chondrogenic cells, which can be used as disease models for drug screening, mechanism study, toxicity assay, or other research and drug discovery and development tools. For example, the method comprises exposing an iPSC, an embryonic stem cell, a progenitor cell or a differentiated cell to a composition comprising a transducible material to reprogram the iPSC, embryonic stem cell, progenitor cell or differentiated cell to a transplantable chondrogenic cell; transplanting the transplantable chondrogenic cell into a biological sample or a biological organism; developing the biological sample or biological organism to become a disease model. For another example, the method comprises reprogramming patient-specific cells to chondrogenic cells using a transducible materials; and developing a disease model using patient-specific chondrogenic cells or chondrogenic cell-derived cells. For another example, the method of developing drug screening or toxicity models comprises reprogramming somatic cells, progenitor cells, or multipotent cells to chondrogenic cells using a transducible material and using chondrogenic cells and/or chondrogenic cell-derived cells to screen the effects and/or toxicities of different compounds.

Another aspect of the present disclosure relates to a method of developing cell-based therapies for various diseases or conditions involving cartilage damage comprising the step of reprogramming an iPSC, an embryonic stem cell, a progenitor cell or a differentiated cell to a transplantable chondrogenic cell using a transducible material; transplanting the transplantable chondrogenic cell into a biological sample or biological organism; assessing the therapeutic effect of the transplantable chondrogenic cell.

The transplantable cells can be transplanted to a subject in need, and once in the right place, such as in the injury sites, blood clots, or the subchondral bone marrow space, the transplanted chondrogenic cells or their derived cells can regenerate cartilage tissue, preferably hyaline cartilage tissue and repair cartilage damage.

For example, a patient with a condition involving cartilage damage is treated with protein drugs, small molecule drugs, or the combination, through injection into the injured site of his/her articular cartilage, joint or subchondral bone marrow space, generating new chondrogenic cells at the injured sites, blood clots, or the subchondral bone marrow space. The newly generated chondrogenic cells regenerate hyaline cartilage tissue and repair cartilage damage.

Another aspect of the present disclosure relates to a method of identifying a effector domain, wherein the method comprises the steps of covalently linking a test effector domain to a know transduction domain to form a test transducible molecule; exposing the test molecule to a biological sample, and measuring the reprogramming of the biological sample to a chondrogenic sample to indicate whether the test effector domain can exerts a change in the biological sample. It is also contemplated that another aspect of the present disclosure relates to a method of identifying a transducible domain, wherein the method comprises the steps of covalently linking a known effector domain to a test transduction domain to form a test transducible molecule; exposing the test molecule to a biological sample, and measuring the location of the test molecule in or the reprogramming effect of the biological sample to a chondrogenic sample to indicate whether the test transduction domain can transduce the effector domain into the biological sample.

### EXAMPLES

The following examples are provided to better illustrate the claimed invention and are not to be interpreted in any way as limiting the scope of the invention. These specific compositions, materials, and methods are not intended to limit the invention, but merely to illustrate specific embodiments falling within the scope of the invention. One skilled in the art may develop equivalent compositions, materials, and methods without the exercise of inventive capacity and without departing from the scope of the invention. It will be understood that many variations can be made in the procedures herein described while still remaining within the bounds of the invention. It is the intention of the inventors that such variations are included within the scope of the invention.

### Example 1 (reference example). Reprogramming somatic cells to induced pluripotent stem cells (iPSCs)

### 1.a. Preparation of transducible material Oct4-11R, Sox2-11R, K1f4-11R, and cMyc-11R.

A poly-arginine protein transduction domain was fused to the C-terminal of each reprogramming proteins Oct4, Sox2, Klf4 and cMyc through a linker SEQ ID NO. 55 to form a fused protein Oct4-11R, Sox2-11R, K1f4-11R and cMyc-11R respectively (Figure 1A). These poly-arginine fused proteins were expressed in *E. Coli* in inclusion body form, which were then solubilized, refolded, and further purified to render transducible materials Oct4-11R, Sox2-11R, K1f4-11R and cMyc-11R. The protein identities were confirmed by mass spectrometry and Western blot analysis (Figure 1B).

### 1.b. Cell permeability and stability of transducible material Oct4-11R, Sox2-11R, K1f4-11R, and cMyc-11R

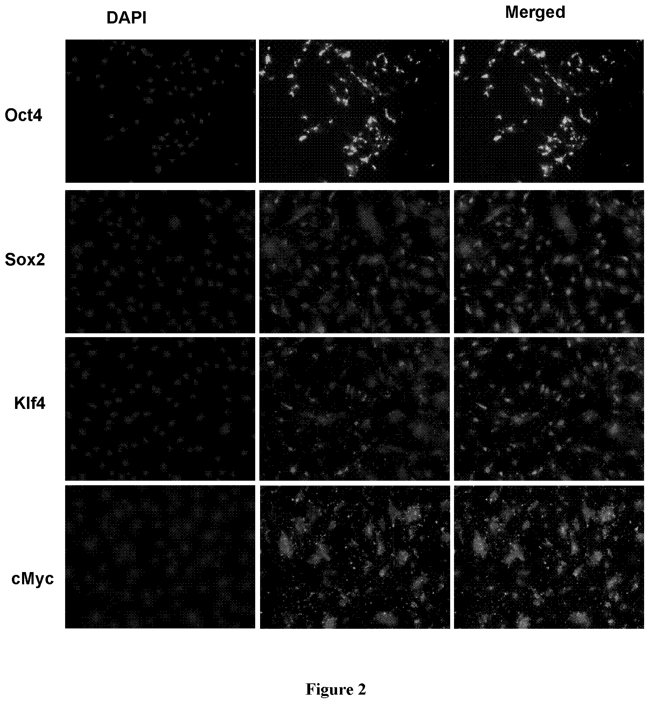

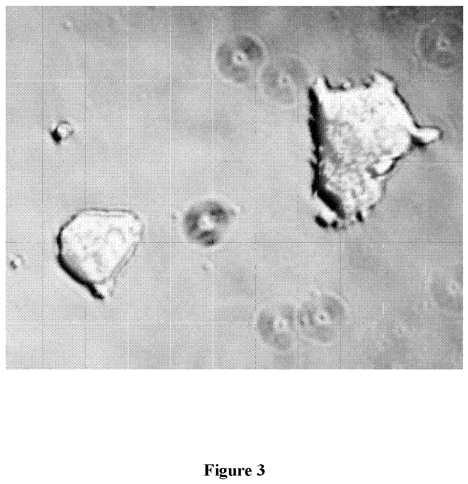

A transducible material (Oct4-11R, Sox2-11R, K1f4-11R, or cMyc-11R) was added to mouse embryonic fibroblast (MEF) cells at various concentrations for 6-72 hours. Cell morphology and protein presence were examined by immunocytochemistry. The transducible materials were found to enter cells at concentrations of 0.5-8 µg/ml within 6 hours, and translocated into nucleus (Figure 2). In addition, the transduced proteins were fairly stable inside of cells for up to 48 hours (Figure 3).

### 1.c. Reprogramming OG2/Oct4-GFP reporter MEF cells.

The protein transduction condition described in paragraph 0047 was used to reprogram OG2/Oct4-GFP reporter MEF cells. Cells were treated in 4 cycles. In each cycle the fibroblasts (initially seeded at the density of 5x10⁴ cells/well in a six-well plate) were first treated with transducible materials Oct4-11R, Sox2-11R, K1f4-11R and cMyc-11R at 8 µg/ml in the mESC growth media supplemented with or without 1 mM valproic acid (VPA, a inhibitor of the enzyme histone deacetylase 1 (HDAC1)) for overnight, followed by changing to the same media without the transducible material and VPA, and culturing for additional 36 hours before the next cycle of the treatment. After completing repeated protein transduction of a transducible material, the treated cells were transferred onto irradiated MEF feeder cells and kept in mESC growth media until colonies emerged around day 30-35 (Figure 4A). 3 GFP+ colonies per 5x10⁴ cells were obtained when the cells were transduced with Oct4-11R, Sox2-11R, Klf4-1 1R, and cMyc-11R and treated with VPA, and 1 GFP+ colony per 5x10⁴ cells were obtained when the cells were transduced with Oct4-11R, Sox2-11R, or K1f4-11R respectively and treated with VPA. Those initial GFP+ colonies were subsequently passaged under conventional mESC growth conditions to yield piPS cells, and were further characterized.

The generated murine piPS cells have been stably expanded for over twenty passages, and were morphologically indistinguishable to classic mES cells, forming compact domed small colonies (Figures 4B and 4C). They expressed typical pluripotency markers examined by immunocytochemistry and staining, including ALP (Figure 4D), Oct4, Nanog, Sox2, and SSEA1 (Figure 4E). RT-PCR analysis confirmed endogenous gene expression of these pluripotency markers and additional pluripotency genes (Figure 4F). A single cell survival assay also demonstrated that piPS cells clonally expanded efficiently as Oct4-positive colonies in feeder-free and N2/B27-chemically defined conditions. Furthermore, bisulphite genomic sequencing analyses of the Oct4 promoter revealed that it was demethylated in piPS cells similarly to the mES cells, while the MEFs' Oct4 promoter was hypermethylated (Figure 4G). This result further suggests a reactivation of the pluripotency transcription program in these piPS cells.

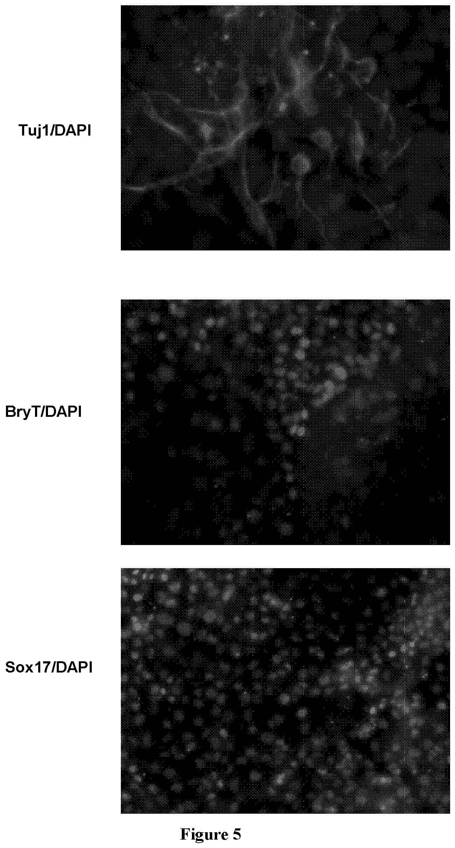

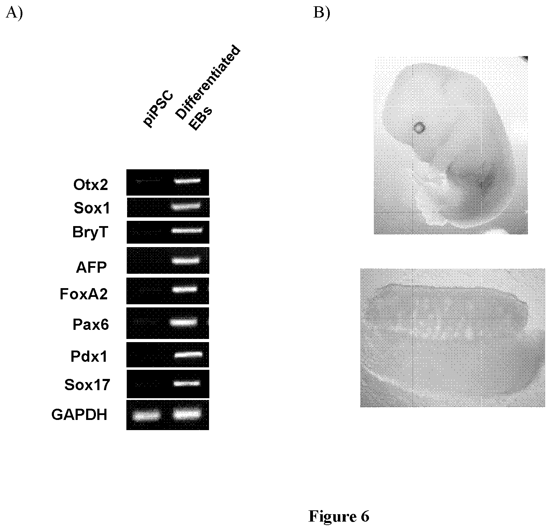

To examine the developmental potential of piPS cells, standard *in vitro* differentiation using embryoid bodies (EB) or monolayer chemically defined step-wise differentiation, as well as in vivo chimerism assays were performed. piPS cells efficiently formed EB in suspension, and differentiated into cells in the three primary germ layers, including primitive endoderm (AFP, Sox17), foregut endoderm (FoxA2), pancreatic cells endoderm (PDX1, Pax6), mesoderm (Brachyury), and neural (Sox1) and neuronal cells (βIII-tubulin)-ectoderm (Figures 5 and 6A). These piPS cells efficiently incorporated into the inner cell mass of a blastocyst following aggregation with an 8-cell embryo, and led to chimerism with germline contribution (Figure 6B) *in vivo* after the aggregated embryos were transplanted into mice, as suggested by observation of Oct4-GFP+ cells in the gonad tissue in 2 out of 7 embryos (Figure 6B bottom). These *in vitro* and *in vivo* characterizations collectively confirm that the purified transduction material Oct4-11R, Sox2-11R, K1f4-11R, and cMyc-11R are able to reprogram MEFs to piPS cells, which are morphologically and functionally similar to conventional mES cells.

### Example 2 (reference example). Reprogramming of liver and pancreatic exocrine cells to insulin-producing beta cells by transducible materials His6-Ngn3-11R, His6-PDX1-11R and His6-MafA-11R in mouse.

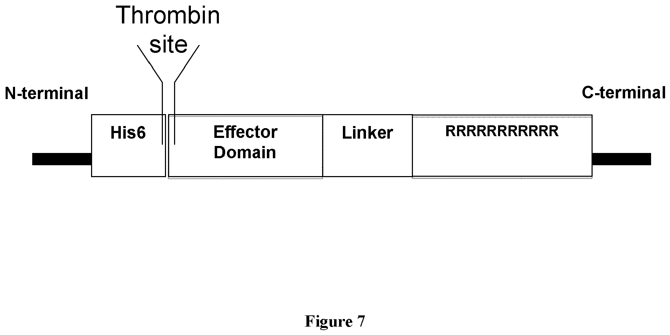

A poly-arginine protein transduction domain was fused respectively to the C-terminal of each reprogramming protein (Ngn3, PDX1 and MafA) through a linker (**SEQ ID NO: 55**) to form His6-Ngn3-11R, His6-PDX1-11R and His6-MafA-11R respectively (Figure 7). His6 (**SEQ ID NO: 59**) was included to facilitate protein purification. These poly-arginine fused proteins were expressed in *E. Coli* in inclusion body form, which were then solubilized, refolded, and further purified to prepare transducible materials His6-Ngn3-11R, His6-PDX1-11R and His6-MafA-11R. The protein identities were confirmed by mass spectrometry and Western blot analysis.

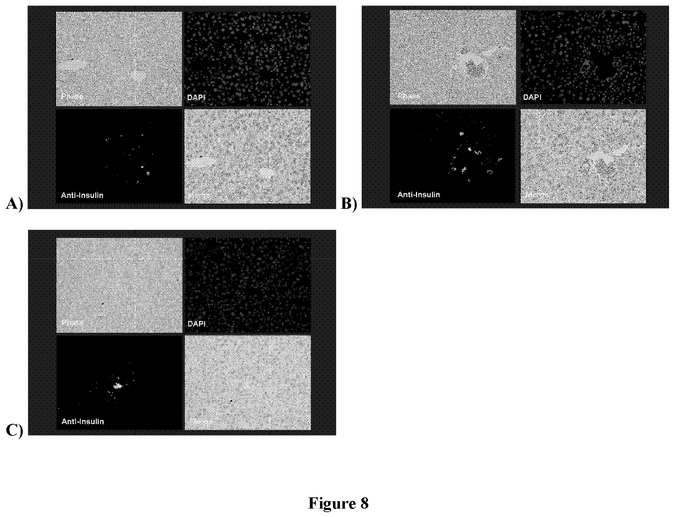

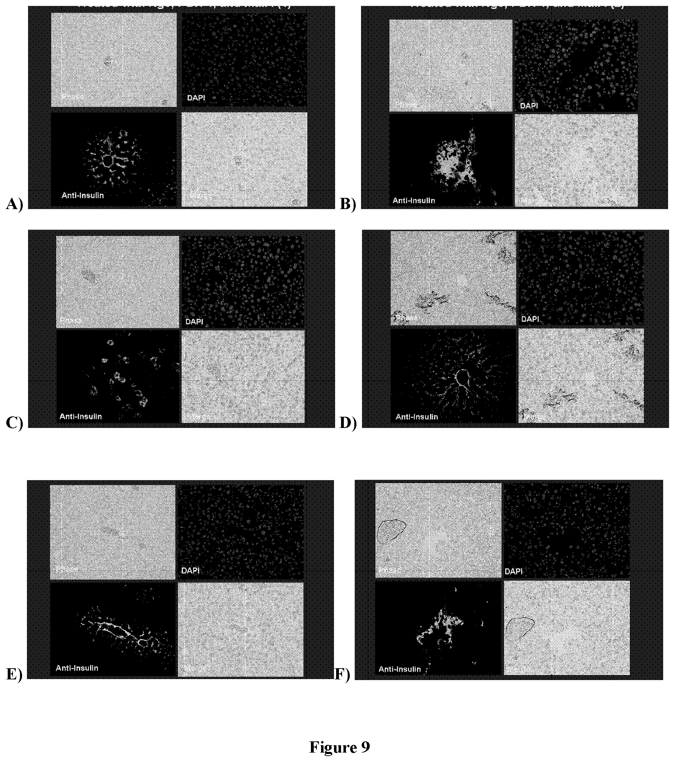

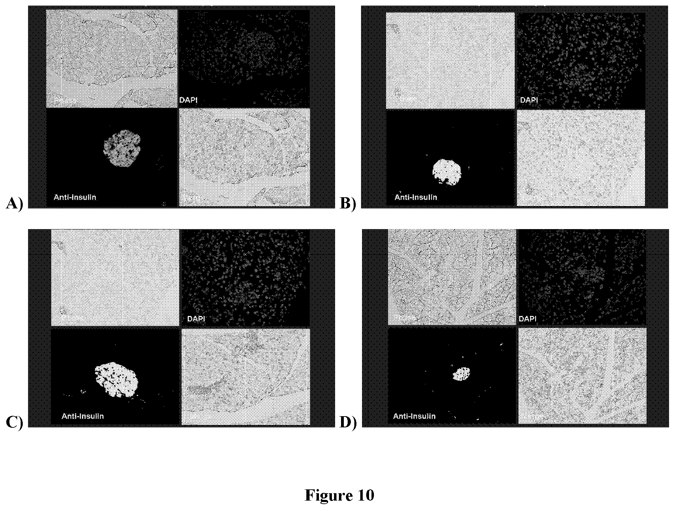

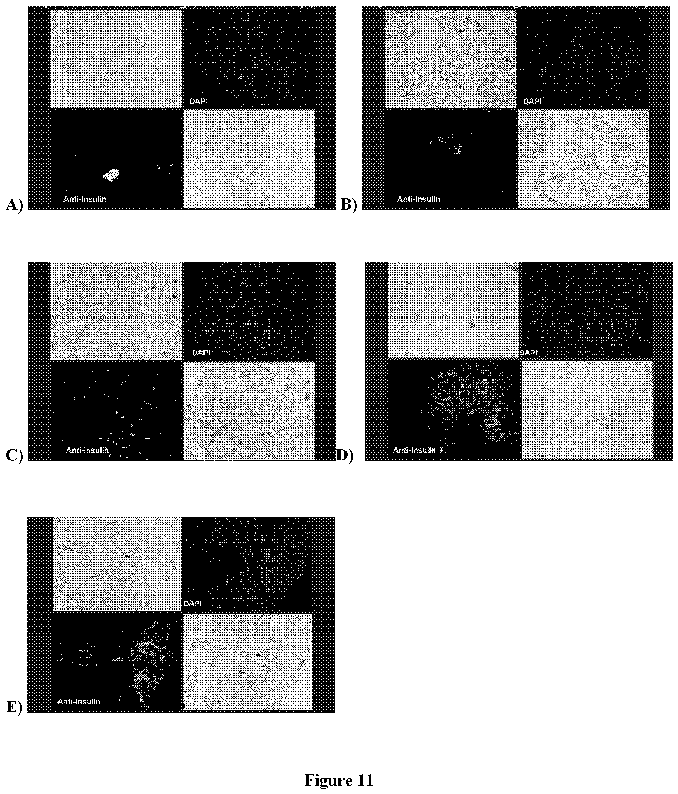

Six CD-1 mice (Charles River Laboratory) were divided into two groups: the treatment group and the control group. Transducible material His6-Ngn3-11R (1mg/kg), His6-PDX1-11R (1mg/kg), and His6-MafA-11R (1mg/kg) were injected into each mouse by intraperitoneal (IP) in treatment group (Mouse-4, Mouse-5 and Mouse-6) and BSA (1mg/kg) was injected into each mouse in the control group (Mouse-1, Mouse-2 and Mouse-3). There was no Greenish-brown or Yellow aspirate when needle penetrated into each mouse peritonea. Injections were repeated every day for 7 days. Mice of both treatment and control group were sacrificed on the 3rd day after the completion of all injections. The mouse liver and pancreas were washed with IX PBS and fixed by 4% paraformaldehyde for overnight. Then the liver and pancreatic tissues were processed by standard Paraffin *Embedding* protocol. The Tissue sections, 5-micro in thickness, were prepared routinely with histology microtomes and mounted on standard histology glass slides. The wax in tissues was dissolved by xylene during processing of tissue sections. Tissue sectioning and histologic and immunohistochemical staining were performed using routine methods. For indirect fluorescent-antibody (IFA) assay, the slides were blocked with 0.05% Tween-20 (TBST) and 3% BSA for 1 hour at RT and were incubated with mouse anti-insulin antibody (Invitrogen) at 4°C overnight. The slides were washed three times with PBS for 15 minutes at RT and incubated with fluorescein isothiocyanate (FITC) conjugated swine anti-mouse antibody (KPL) for 2 hours at RT. Same concentration of Mouse IgG was used as isotype control. Anti-DAPI antibody was added to slides as a nuclear marker. The slides were washed as before and mounted with aqueous mounting media (Biomeda, Foster City, CA). Endothelial markers were identified under the microscope (Olympus BX51, San Diego, CA) and merged cells were analyzed by Microsuite Biological Suite program (Olympus BX51, San Diego, CA) (Figures 8-11). The results showed that the treatment group had more insulin-producing cells (Figure 9) in livers comparing to the control group (Figure 8). The pancreas of the control group showed a cluster of insulin-producing cells (Figure 10), while the pancreas of the treatment group showed insulin-producing cells in bigger area (Figure 11). Therefore, the results showed that treatment of transducible materials His6-Ngn3-11R, His6-PDX1-11R, and His6-MafA-11R converted liver and/or pancreas cells to insulin-producing cells.

### Example 3 (reference example). Reprogramming of T cells and programs them to Treg cells using transducible material Foxp3.

A poly-arginine protein transduction domain was fused to the C-terminal of each reprogramming protein Foxp3 through a linker (SEQ ID NO: 55) to form His6-Foxp3-11R (Figure 7). His6 (SEQ ID NO: 59) was included to facilitate protein purification. The poly-arginine fused protein was expressed in *E. Coli* in inclusion body form, which were then solubilized, refolded, and further purified to prepare transducible materials His6-Foxp3-11R. The protein identities were confirmed by Western blot analysis.

100ml of healthy human blood was collected from a donor and the peripheral blood mononuclear cells (PBMCs) were isolated by density-gradient centrifugation using Histopaque-1077 (Sigma-Aldrich, St Louis, MO). CD14+ monocytes were removed by magnetic bead selection (Miltenyi Biotec, Auburn, CA).Briefly, 108 PBMCs were incubated with 200 µL anti-CD 14 microbeads (Miltenyi Biotec) in ice for 30 minutes. The cells were washed with cold IX PBS with 2% FCS and centrifuged at 300g for 10 minutes and then resuspended in IX PBS with 2% FCS. The cell suspension was applied to the magnetic column and unbinding cells were passed through by washing 3 times with IX PBS with 2% FCS. The PBMC/mono- were harvested by centrifuged at 300g for 10 minutes.

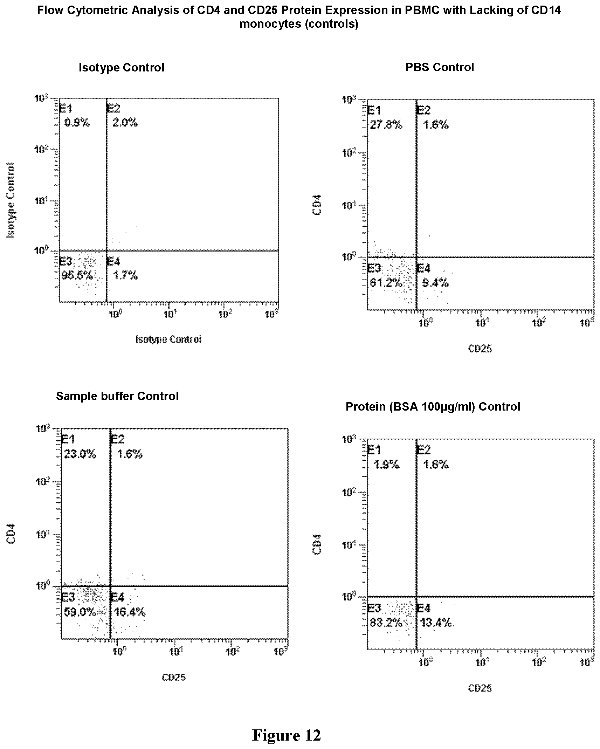

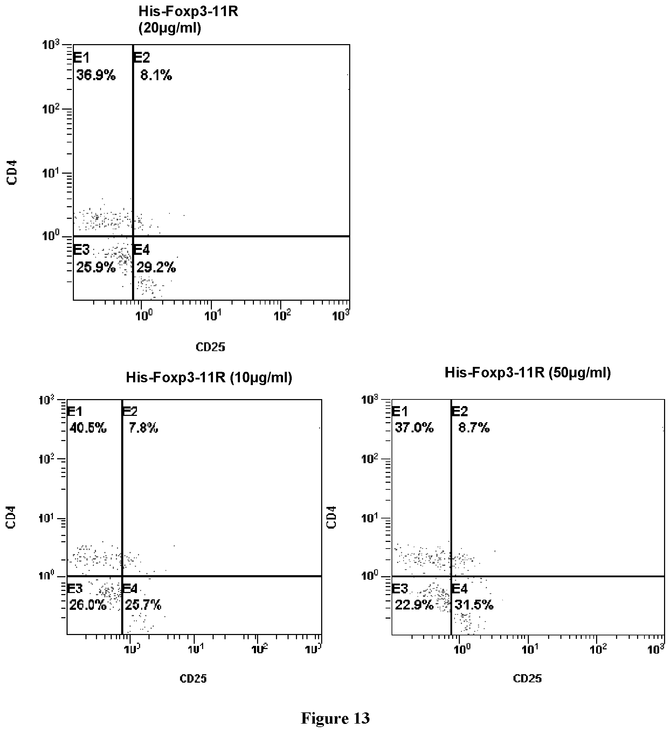

The PBMC/mono- were cultured in 6-well plates (Becton Dickinson, Gaithersburg, MD) supplemented with 10% FBS, nonessential amino acids, 2 mM glutamine, 1 mM sodium pyruvate, 25 mM HEPES, 200 units/ml penicillin, and streptomycin at 37 °C and 5% CO₂. After 1 hour of culture, His6-Foxp3-11R (10µg/ml, 20 µg/ml, or 50 µg/ml) was added to the cells. BSA (100µg/ml) was added to another well as control. Same concentration of His6-Foxp3-11R or BSA was added after cultured for two days. After 5 days of culture, the cells were washed with PBS twice. The cells were re-suspended in 100 µL diluted and added rabbit anti-human CD25 for 90 minutes. The cells were washed three time with cold 1XPBS supplied 2% FBS and then the conjugated-PE mouse anti-human CD4 as well as conjugated-FITC goat anti-rabbit IgG were added to the cells for 60 minutes in ice. Conjugated-PE mouse IgG and rabbit IgG were incubated with another group cells as Isotype control. The cells were washed with PBS for flow cytometric analysis using a Beckman Coulter FC500 cytometer with Cytomics CXP software (Beckman Coulter, Fullerton, CA) (Figures 12 and 13). The results showed that the CD4+CD25+T cells (Treg cells) have dramatically increased with treatment of transducible material His6-Foxp3-11R, and the increase is protein-dose dependent.

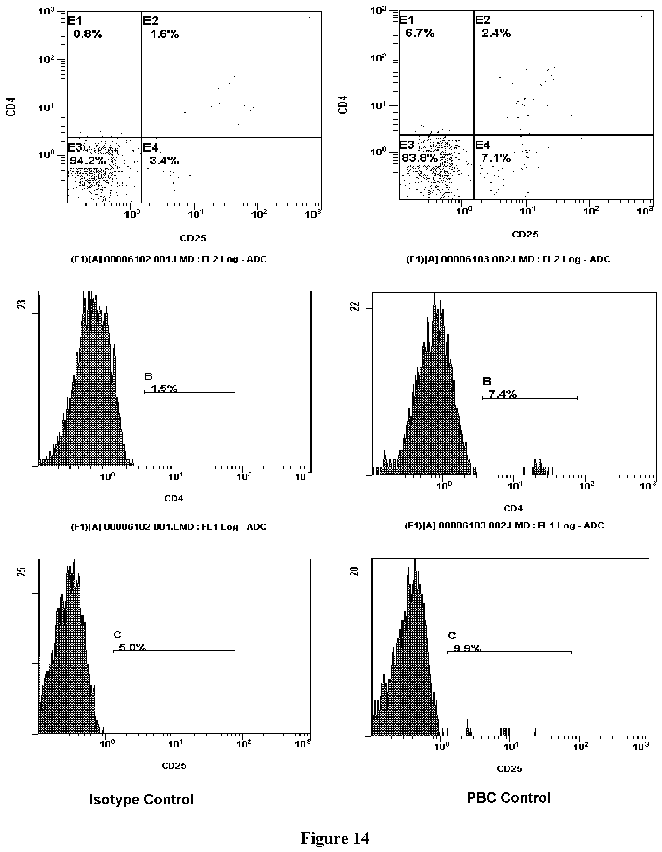

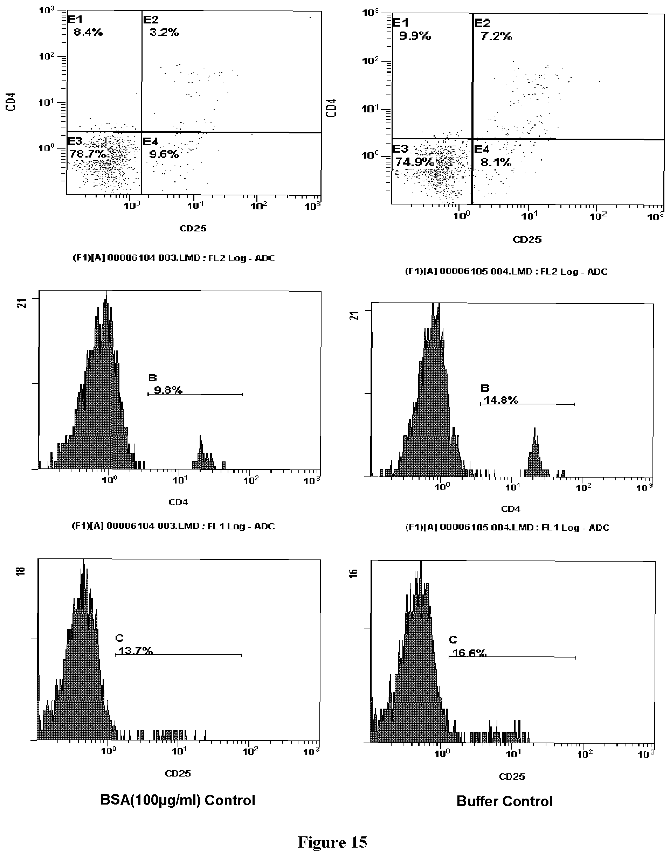

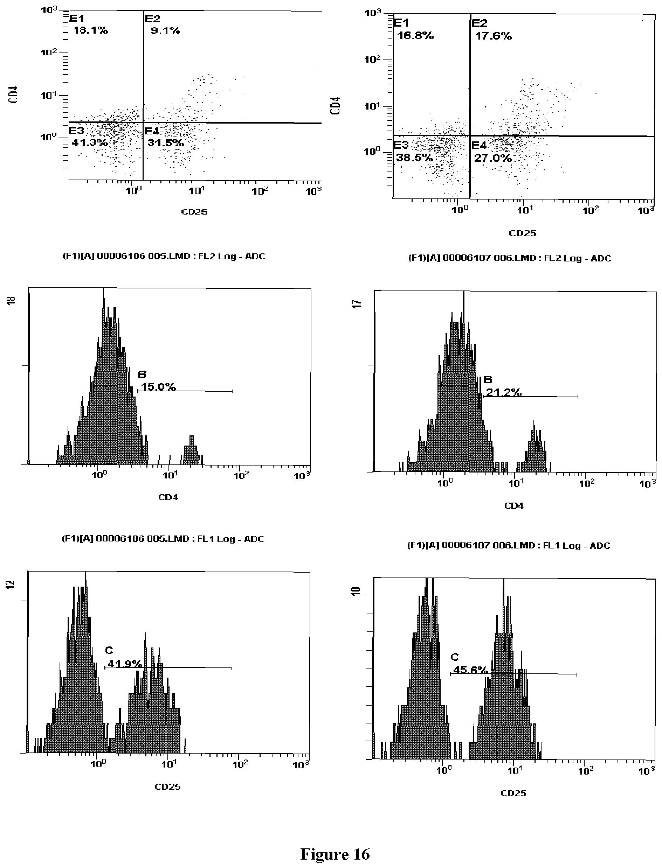

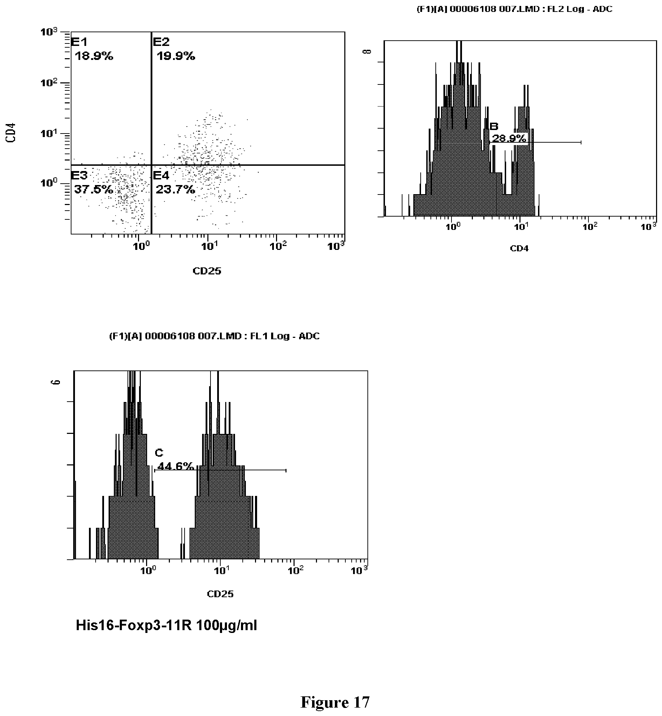

100ml of healthy human blood was collected from a donor and the peripheral blood mononuclear cells (PBMCs) were isolated by density-gradient centrifugation using Histopaque-1077 (Sigma-Aldrich, St Louis, MO). The PBMC/mono⁻were cultured in 6-well plates (Becton Dickinson, Gaithersburg, MD) supplemented with 10% FBS, nonessential amino acids, 2 mM glutamine, 1 mM sodium pyruvate, 25 mM HEPES, 200 units/ml penicillin, and streptomycin at 37°C and 5% CO2. After 1 hour of culture, Foxp3 (10)µg/ml, 50 µg/ml, 100 µg/ml) were added to the cells. BSA (100µg/ml) was added to another well as control. Same concentration of the Foxp3 or BSA was added after cultured two days. Following 5 days of culture, the cells were washed with PBS twice. The cells were re-suspended in 100 µL diluted and added rabbit anti-human CD25 for 90 minutes. The cells were washed three time with cold 1XPBS supplied 2% FBS and then the conjugated-PE mouse anti-human CD4 as well as conjugated-FITC goat anti-rabbit IgG were added to the cells for 60minutes in ice. Conjugated-PE mouse IgG and rabbit IgG were incubated with another group cells as Isotype control. The cells were washed with PBS for flow cytometric analysis using a Beckman Coulter FC500 cytometer with Cytomics CXP software (Beckman Coulter, Fullerton, CA) (Figures 14-17). The results showed that the CD4+CD25+T cells (Treg cells) have dramatically increased with treatment of transducible material His6-Foxp3-11R, and the increase is protein-dose dependent.

### Example 4. Reprogramming of bone marrow derived MSC and reprograms them to chondrogenic cells by supercharged Sox9.

A bioactive supercharged Sox9 (scSox9, also referred to as scGFP-Sox9-11R, the sequence of which is shown in Figure 22(n)) was generated by fusing Sox9 with a super positively charged green fluorescence protein (scGFP) using molecular engineering technology. Briefly, the cDNA sequence encoding scSox9 was first optimized for high level protein expression in E. coli and then synthesized. Next, the gene was subcloned into vector pET-15b and this protein expression plasmid was then transformed into BL21 (DE3) competent cells. Finally the fusion protein was produced, refolded, and purified from E. coli culture.

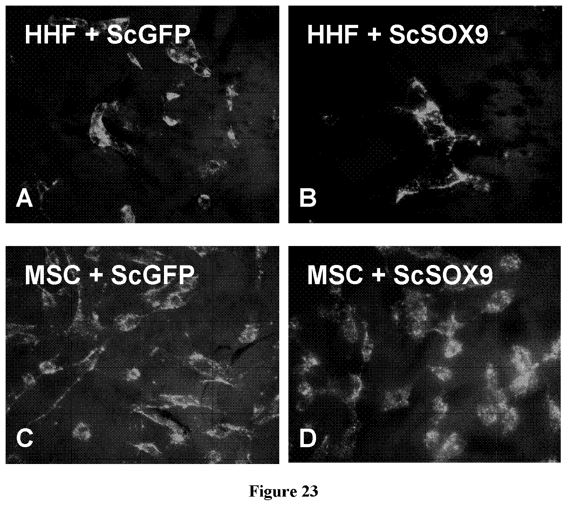

Human skin fibroblast cell line (HHF) and human bone marrow derived mesenchymal stem cells (MSC) at passage 5 were cultured with 10 µg/ml scSox9 or with 10 µg/ml scGFP (negative control) in monolayer or in cell aggregate in DMEM medium at 37°C. After one hour incubation with scSox9, cells were washed and viewed under fluorescent microscope. Both HHF cells and MSC showed intracellular expression of green fluorescence, indicating entry of scSox9 into these cells (Figure 23).

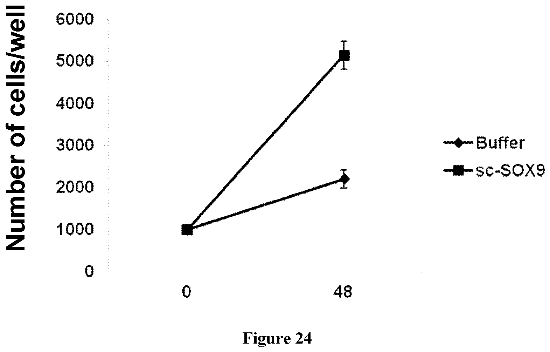

MSC were cultured in DMEM containing 10% FBS and low glucose (1.5g/l) with addition of scSox9 or buffer. The number of scSox9 treated MSC was increased two folds after 72 hours in culture compared with buffer treated MSC (Figure 24). The results showed that scSox9 was able to induce MSC proliferation and differentiation with no requirement of additional growth factors.

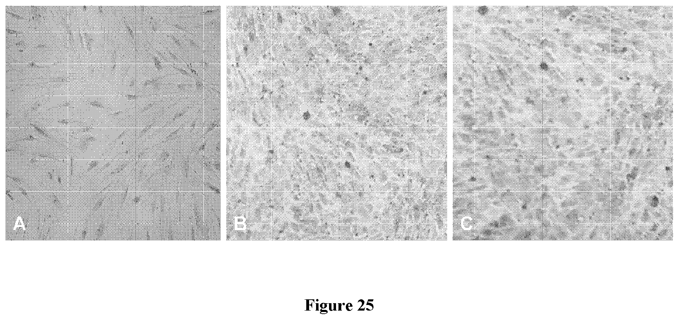

Next, the differentiation of MSC into chondrocytes was investigated. MSC were cultured in DMEM containing 1% FBS and high glucose (4.5 g/l) and 10 µg/ml of scSox9 was added. After 24 hours, culture medium was changed to DMEM containing 0% FBS and high glucose with no additional scSox9 was added. Culture was maintained for 14 days. As early as 48 hours, scSox9 treated MSC started to change morphology into chondrocyte-like cells and this morphology maintained for 21 days in culture. These morphologically changed cells stained positive for toluidine blue, which demonstrated production of aggrecan, which is a major component of proteoglycan in articular cartilage matrix, and chondrocytes' functions (Figure 25). The results showed that scSox9 induced MSC to differentiate into chondrocytes.

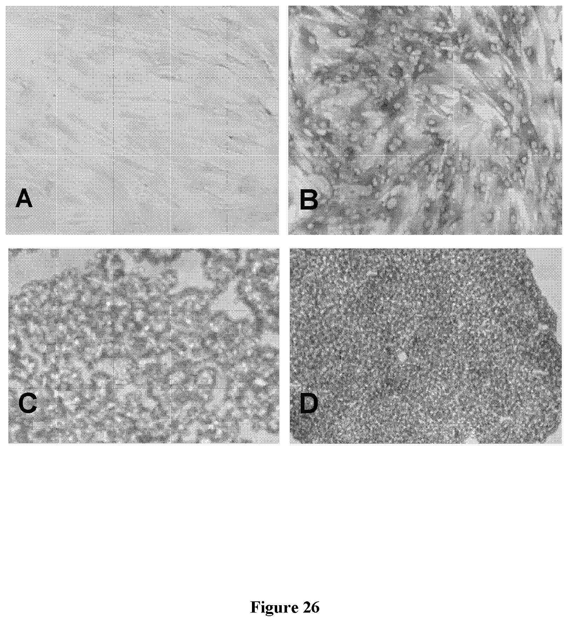

Furthermore, scSox9 induced collagen type II expression and downregulated collagen type I and type X production (Figures 26-27). Human bone marrow derived MSC was cultured with 10 µg/ml of scGFP (Figure 26A and C) or scSox9 (Figure 26 B and D) in monolayer (Figure 26A and B) or aggregate (Figure 26C and D) for chondrogenesis. At day 14, aggregates were harvested and snap-frozen. Cryostat sections were stained with a mouse anti-human collage type II monoclonal antibody (Immunoperoxidase staining). As shown in Figure 26, MSC cultured with scGFP only showed poorly formed aggregate, but MSC cultured with scSox9 were positive for collagen II, and showed good formation of aggregate.

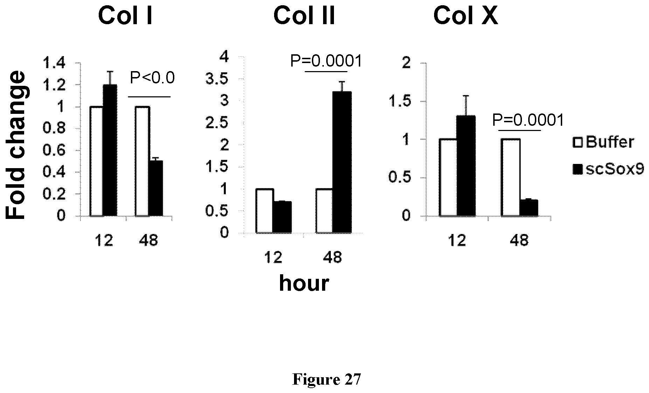

MSC were cultured with DMEM containing 1% FBS and high glucose (4.5g/l) with addition of buffer only or 10 µg/ml of scSox9. At 12 hours and 48 hours after the treatment, RNA was extracted and RT-PCR was performed with TaqMan probe based analysis assay for collagen (Col) type I, II and X mRNA expression. Expression as relative to GAPDH. As shown in Figure 27, scSox9 induced increased collagen type II but decreased collagen type I and type X expression.

Type X collagen is typically expressed in hypertrophic chondrocytes, and Type I collagen in fibrocartilage, whereas the superior hyaline cartilage expresses Type II collagen. The data indicated that while promoting chondrogenesis, scSox9 can concomitantly repress chondrocyte hypertrophy and inhibit osteogenesis. Most importantly, one time addition of scSox9 was sufficient to induce MSC differentiation into chondrocytes and maintained the chondrocyte phenotype.

These in vitro data demonstrated that scSox9 is able to enter bone marrow derived MSC and induce chondrogenesis and maintain chondrocyte phenotype.

## Claims

1. An *in vitro* method of reprogramming a mesenchymal stem cell (MSC), comprising:
exposing the MSC to a transducible material comprising Sox9 polypeptide and a transduction domain linked to the Sox9 polypeptide covalently, non-covalently or via a linker, wherein the transduction domain is a supercharged GFP, and wherein the MSC is reprogrammed to change to a chondrogenic cell.

2. A transducible material comprising Sox9 polypeptide and a transduction domain linked to the Sox9 polypeptide covalently, non-covalently or via a linker, wherein the transduction domain is a supercharged GFP; for use in therapy to reprogram an MSC, comprising:
exposing the MSC to the transducible material; wherein the MSC is reprogrammed to change to a chondrogenic cell.

3. The method according to claim 1, or transducible material for use according to claim 2, wherein the MSC is a human MSC.

4. The method according to claim 1, or transducible material for use according to claim 2, wherein the linker has the amino acid sequence set forth in SEQ ID NO: 55.

5. The method according to claim 1, or transducible material for use according to claim 2, wherein the transducible material further comprises one or more motifs that do not interrupt the function of Sox9 polypeptide or the transduction domain.

6. The method or transducible material for use according to claim 5, wherein the motif is covalently linked to the Sox9 polypeptide or the transduction domain.

7. The method or transducible material for use according to claim 5, wherein the motif has the amino acid sequence set forth in SEQ ID NO: 59.

8. The method of claim 1, or transducible material for use according to claim 2, wherein the Sox9 polypeptide has the amino acid sequence set forth in SEQ ID NO: 69.

9. The method of claim 1 or transducible material for use according to claim 2, wherein the transducible material comprises a polypeptide selected from the group consisting of: HA2-Supercharged GFP-Sox9 and scGFP-Sox9-11R.

10. A transducible material comprising Sox9 polypeptide and a transduction domain linked to the Sox9 polypeptide covalently, non-covalently or via a linker; for use in therapy by treating a condition involving cartilage damage in a biological organism, wherein the transduction domain is a supercharged GFP, optionally wherein the condition involving cartilage damage is joint disorder, osteoarthritis, cartilage injury, traumatic rupture or detachment, achondroplasia, costochondritis, spinal disc herniation, relapsing polychonritis, tumor, chondroma, chondrosarcoma, or peomorphic adenoma.

11. An "in vitro" method of developing cell-based therapies for diseases or conditions involving cartilage damage comprising:
reprogramming an MSC to a transplantable chondrogenic cell by exposing the MSC to a transducible material comprising Sox9 polypeptide and a transduction domain linked to the Sox9 polypeptide covalently, non-covalently or via a linker, wherein the transduction domain is a supercharged GFP;
transplanting the transplantable chondrogenic cell into a biological sample; and
assessing the therapeutic effects of the transplantable chondrogenic cell;

12. A transducible material comprising Sox9 polypeptide and a transduction domain linked to the Sox9 polypeptide covalently, non-covalently or via a linker; for use in therapy to reprogram an MSC, in a method of developing cell-based therapies for diseases or conditions involving cartilage damage comprising:
reprogramming the MSC to a transplantable chondrogenic cell by exposing the MSC to the transducible material;
transplanting the transplantable chondrogenic cell into a biological organism; and
assessing the therapeutic effects of the transplantable chondrogenic cell,
wherein the transduction domain is a supercharged GFP.

## Patentansprüche

1. In-vitro-Verfahren zur Umprogrammierung einer mesenchymalen Stammzelle (MSC), umfassend:
Inkontaktbringen der MSC mit einem transduzierbaren Material, das Sox9-Polypeptid und eine mit dem Sox9-Polypeptid kovalent, nicht kovalent oder über einen Linker verknüpfte Transduktionsdomäne umfasst, wobei es sich bei der Transduktionsdomäne um ein Supercharged-GFP (GFP mit sehr hoher Nettoladung) handelt und wobei die MSC umprogrammiert wird, so dass sie sich zu einer chondrogenen Zelle ändert.

2. Transduzierbares Material, umfassend Sox9-Polypeptid und eine mit dem Sox9-Polypeptid kovalent, nicht kovalent oder über einen Linker verknüpfte Transduktionsdomäne, wobei es sich bei der Transduktionsdomäne um ein Supercharged-GFP handelt, zur Verwendung bei einer Therapie zum Umprogrammieren einer MSC, umfassend:
Inkontaktbringen der MSC mit dem transduzierbaren Material, wobei die MSC umprogrammiert wird, so dass sie sich zu einer chondrogenen Zelle ändert.

3. Verfahren nach Anspruch 1 oder transduzierbares Material zur Verwendung nach Anspruch 2, wobei es sich bei der MSC um eine menschliche MSC handelt.

4. Verfahren nach Anspruch 1 oder transduzierbares Material zur Verwendung nach Anspruch 2, wobei der Linker die Aminosäuresequenz gemäß SEQ ID NO: 55 aufweist.

5. Verfahren nach Anspruch 1 oder transduzierbares Material zur Verwendung nach Anspruch 2, wobei das transduzierbare Material ferner ein oder mehrere Motive umfasst, die die Funktion von Sox9-Polypeptid oder der Transduktionsdomäne nicht stören.

6. Verfahren oder transduzierbares Material zur Verwendung nach Anspruch 5, wobei das Motiv mit dem Sox9-Polypeptid oder der Transduktionsdomäne kovalent verknüpft ist.

7. Verfahren oder transduzierbares Material zur Verwendung nach Anspruch 5, wobei das Motiv die Aminosäuresequenz gemäß SEQ ID NO: 59 aufweist.

8. Verfahren nach Anspruch 1 oder transduzierbares Material zur Verwendung nach Anspruch 2, wobei das Sox9-Polypeptid die Aminosäuresequenz gemäß SEQ ID NO: 69 aufweist.

9. Verfahren nach Anspruch 1 oder transduzierbares Material zur Verwendung nach Anspruch 2, wobei das transduzierbare Material ein Polypeptid umfasst, das aus der Gruppe bestehend aus HA2-Supercharged-GFP-Sox9 und scGFP-Sox9-11R ausgewählt ist.

10. Transduzierbares Material, umfassend Sox9-Polypeptid und eine mit dem Sox9-Polypeptid kovalent, nicht kovalent oder über einen Linker verknüpfte Transduktionsdomäne, zur therapeutischen Verwendung bei der Behandlung eines Leidens, bei dem eine Knorpelschädigung vorliegt, wobei es sich bei der Transduktionsdomäne um ein Supercharged-GFP handelt, gegebenenfalls wobei es sich bei dem Leiden, bei dem eine Knorpelschädigung vorliegt, um Gelenkerkrankung, Osteoarthritis, Knorpelverletzung, traumatische Ruptur oder Ablösung, Achondroplasie, Costochondritis, Bandscheibenvorfall, rezidivierende Polychondritis, Tumor, Chondrom, Chondrosarkom oder pleomorphes Adenom handelt.

11. "In-vitro"-Verfahren zur Entwicklung von Therapien auf Zellbasis für Krankheiten oder Leiden, bei denen eine Knorpelschädigung vorliegt, umfassend:
Umprogrammieren einer MSC zu einer transplantierbaren chondrogenen Zelle durch Inkontaktbringen der MSC mit einem transduzierbaren Material, das Sox9-Polypeptid und eine mit dem Sox9-Polypeptid kovalent, nicht kovalent oder über einen Linker verknüpfte Transduktionsdomäne umfasst, wobei es sich bei der Transduktionsdomäne um ein Supercharged-GFP handelt;
Transplantieren der transplantierbaren chondrogenen Zelle in eine biologische Probe; und
Beurteilen der therapeutischen Wirkungen der transplantierbaren chondrogenen Zelle.

12. Transduzierbares Material, umfassend Sox9-Polypeptid und eine mit dem Sox9-Polypeptid kovalent, nicht kovalent oder über einen Linker verknüpfte Transduktionsdomäne, zur therapeutischen Verwendung zum Umprogrammieren einer MSC, in einem Verfahren zur Entwicklung von Therapien auf Zellbasis für Krankheiten oder Leiden, bei denen eine Knorpelschädigung vorliegt, umfassend:
Umprogrammieren der MSC zu einer transplantierbaren chondrogenen Zelle durch Inkontaktbringen der MSC mit dem transduzierbaren Material;
Transplantieren der transplantierbaren chondrogenen Zelle in einen biologischen Organismus; und
Beurteilen der therapeutischen Wirkungen der transplantierbaren chondrogenen Zelle,
wobei es sich bei der Transduktionsdomäne um ein Supercharged-GFP handelt.

## Revendications

1. Procédé *in vitro* de reprogrammation d'une cellule souche mésenchymateuse (CSM), comprenant :
l'exposition de la CSM à un matériau transductible comprenant le polypeptide Sox9 et un domaine de transduction lié au polypeptide Sox9 de façon covalente, de façon non covalente ou par l'intermédiaire d'un lieur, dans lequel le domaine de transduction est une GFP superchargée, et dans lequel la CSM est reprogrammée pour devenir une cellule chondrogénique.

2. Matériau transductible comprenant un polypeptide Sox9 et un domaine de transduction lié au polypeptide Sox9 de façon covalente, de façon non covalente ou par l'intermédiaire d'un lieur, dans lequel le domaine de transduction est une GFP superchargée ; pour utilisation en thérapie pour reprogrammer une CSM, comprenant :
l'exposition de la CSM au matériau transductible ; la CSM étant reprogrammée pour devenir une cellule chondrogénique.

3. Procédé selon la revendication 1, ou matériau transductible pour utilisation selon la revendication 2, dans lequel la CSM est une CSM humaine.

4. Procédé selon la revendication 1, ou matériau transductible pour utilisation selon la revendication 2, dans lequel le lieur a la séquence d'acides aminés décrite dans SEQ ID NO: 55.

5. Procédé selon la revendication 1, ou matériau transductible pour utilisation selon la revendication 2, dans lequel le matériau transductible comprend en outre un ou plusieurs motifs qui n'interrompent pas la fonction du polypeptide Sox9 ou le domaine de transduction.

6. Procédé ou matériau transductible pour utilisation selon la revendication 5, dans lequel le motif est lié de façon covalente au polypeptide Sox9 ou au domaine de transduction.

7. Procédé ou matériau transductible pour utilisation selon la revendication 5, dans lequel le motif a la séquence d'acides aminés décrite dans SEQ ID NO: 59.

8. Procédé de la revendication 1, ou matériau transductible pour utilisation selon la revendication 2, dans lequel le polypeptide Sox9 a la séquence d'acides aminés décrite dans SEQ ID NO: 69.

9. Procédé de la revendication 1 ou matériau transductible pour utilisation selon la revendication 2, dans lequel le matériau transductible comprend un polypeptide choisi dans le groupe constitué de : HA2-GFP superchargée-Sox9 et scGFP-Sox9-11R.

10. Matériau transductible comprenant un polypeptide Sox9 et un domaine de transduction lié au polypeptide Sox9 de façon covalente, de façon non covalente ou par l'intermédiaire d'un lieur ; pour utilisation en thérapie par traitement d'une affection impliquant des lésions du cartilage dans un organisme biologique, dans lequel le domaine de transduction est une GFP superchargée, facultativement dans lequel l'affection impliquant des lésions du cartilage est un trouble articulaire, l'arthrose, une lésion du cartilage, une rupture traumatique ou un détachement, l'achondroplasie, la costochondrite, une hernie discale, une polychondrite récurrente, une tumeur, un chondrome, un chondrosarcome ou un adénome pléomorphe.

11. Procédé "in vitro" de développement de thérapies à base de cellules pour des maladies ou affections impliquant des lésions du cartilage comprenant :
la reprogrammation d'une CSM en cellule chondrogénique transplantable par exposition de la CSM à un matériau transductible comprenant le polypeptide Sox9 et un domaine de transduction lié au polypeptide Sox9 de façon covalente, de façon non covalente ou par l'intermédiaire d'un lieur, dans lequel le domaine de transduction est une GFP superchargée ;
transplantation de la cellule chondrogénique transplantable dans un échantillon biologique ; et
évaluation des effets thérapeutiques de la cellule chondrogénique transplantable.

12. Matériau transductible comprenant le polypeptide Sox9 et un domaine de transduction lié au polypeptide Sox9 de façon covalente, de façon non covalente ou par l'intermédiaire d'un lieur ; pour utilisation en thérapie pour reprogrammer une CSM, dans un procédé de développement de thérapies à base de cellules pour des maladies ou affections impliquant des lésions du cartilage comprenant :
la reprogrammation de la CSM en cellule chondrogénique transplantable par exposition de la CSM au matériau transductible ;
la transplantation de la cellule chondrogénique transplantable dans un organisme biologique ; et
l'évaluation des effets thérapeutiques de la cellule chondrogénique transplantable, dans lequel le domaine de transduction est une GFP superchargée.
